# EUROPÄISCHE PATENTSCHRIFT

(11) **EP 0 767 732 B1**
(45) Veröffentlichungstag und Bekanntmachung des Hinweises auf die Patenterteilung: **04.12.2002**
(21) Anmeldenummer: 95925799.9
(22) Anmeldetag: 30.06.1995
(51) Int. Cl.: B29C 53/26

(54) **VORRICHTUNG UND VERFAHREN ZUR ERZEUGUNG EINER GEWELLTEN BAHN SOWIE ABSORBIERENDES PRODUKT ENTHALTEND EINE GEWELLTE BAHN**
DEVICE AND PROCESS FOR PRODUCING AN UNDULATING STRIP AND ABSORBENT PRODUCT CONTAINING AN UNDULATING STRIP
DISPOSITIF ET PROCEDE DE PRODUCTION D'UNE BANDE STRIEE ET PRODUIT ABSORBANT CONTENANT UNE BANDE STRIEE

(30) Priorität: 30.06.1994 DE 4422956
(43) Veröffentlichungstag der Anmeldung: 16.04.1997
(60) Teilanmeldung: 01115950.6
(73) Patentinhaber: Hakle-Kimberly Deutschland GmbH, 56070 Koblenz-Rheinhafen (DE)
(72) Erfinder: RAIDEL, Maria, D-90451 Nürnberg (DE); ULLMANN, Jan, D-90402 Nürnberg (DE); ASCHENBRENNER, Franz, D-92280 Kastl (DE)
(74) Vertreter: DIEHL GLAESER HILTL & PARTNER
(86) Internationale Anmeldenummer: EP9502550
(87) Internationale Veröffentlichungsnummer: WO96000625

(56) Entgegenhaltungen:
- EP-A- 0 062 495
- EP-A- 0 137 644
- WO-A-86/02543
- FR-A- 854 979
- FR-A- 1 468 101
- US-A- 2 158 087
- US-A- 2 494 431
- US-A- 2 960 145
- US-A- 3 525 337

## Beschreibung

Die Erfindung betrifft eine Vorrichtung und ein Verfahren zur kontinuierlichen Erzeugung einer zumindest in Teilbereichen gewellten Bahn aus dünnem, blattförmigem und zumindest in Querrichtung dehnbarem Material. Des weiteren betrifft die Erfindung ein absorbierendes Produkt, welches eine gewellte Bahn enthält.

Aus der deutschen Auslegeschrift DE 2011802 B2 ist eine Vorrichtung bekannt, die sich mit der Wellung einer Papierbahn befaßt. Das Führungsbett der bekannten Vorrichtung muß wegen der Materialeigenschaften des verarbeiteten Papiers einen geometrisch sehr komplizierten, räumlich gekrümmten Verlauf aufweisen, der einen sehr hohen fertigungstechnischen Aufwand erfordert und somit nachteilhaft ist.

Aus der deutschen Patentschrift DE 2945395 C2 ist eine Formplatte für eine Falteinrichtung einer Biesennähmaschine, bestehend aus einer Grundplatte und einer Mehrzahl von Faltrippen an ihrer Oberseite, die parallel verlaufende, über den einen Rand der Grundplatte hinausragende Abschnitte und auf der Grundplatte radial bzw. fächerförmig auseinanderlaufende Abschnitte aufweisen, bekannt. Die Faltrippen sind einzelne, an der Grundplatte lösbar angebrachte Klingen, die in Rillen in der Grundplatte angeordnet sind, die jeweils in einem Teilabschnitt eine Vertiefung aufweisen, in die vom unteren Rand der Klingen vorstehende Haltelaschen eingreifen. Die bekannte Vorrichtung ermöglicht keine Stabilisierung einer Wellenform. Die entstehenden Wellen werden vielmehr flachgedrückt und durch Vernähung röhrenförmig auf der Ausgangsbahn fixiert.

Eine weitere Einrichtung zum Ausbilden von Längsfalten in einer laufenden Bahn wird in der deutschen Patentschrift DE 3611134 C2 beschrieben. Hier wird ein sehr elastisches, durch Befeuchtung fast plastisch verformbares Papier für z.B. Zigarettenfilter zwischen einer großen Anzahl von ineinander kämmenden Walzen langsam von der Mitte nach außen wellenförmig eingezogen. Diese sehr aufwendige Maschine ist in der dargestellten Anwendungsform jedoch nur für abschnittsweise Wellenbildung gedacht, über die gesamte Bahn laufende Wellen sind wegen der ständigen wechselseitigen Bahnkrümmung nicht herstellbar.

In der deutschen Patentschrift DE 2827495 C2 schließlich wird eine Vorrichtung zum Fördern und Zusammenlegen für eine Materialbahn beschrieben, wobei im wesentlichen der Transport der Bahn bei gleichzeitigem trichterförmigen Einzug mittels strömender Luft Gegenstand der Erfindung ist. Eine exakt wellenförmige Ausbildung der Falten in der Bahn ist mit der bekannten Vorrichtung nicht möglich.

Der vorliegenden Erfindung liegt somit die Aufgabe zugrunde, eine Vorrichtung und ein Verfahren zur Erzeugung einer gewellten Bahn sowie einen absorbierenden Artikel anzugeben, welche die Nachteile des Stands der Technik vermeiden. Diese Aufgabe löst die Erfindung durch die im unabhängigen Anspruch 1 angegebene Vorrichtung, das im unabhängigen Anspruch 30 angegebene Verfahren und die in den unabhängigen Patentansprüchen 53 und 54 angegebenen absorbierenden Artikel. Weitere vorteilhafte Ausgestaltungen, Aspekte und Details der Erfindung ergeben sich aus den abhängigen Ansprüchen, der Beschreibung und der Zeichnung.

Die erfindungsgemäße Vorrichtung weist ein Führungsbett auf, in das fächerförmige Nuten bzw. Rillen eingeschnitten sind, die bei einer vorzugsweise linear zunehmenden Tiefe schließlich zu einem wellenförmigen Querschnitt der gewünschten Abmessungen führen und in die von der Gegenseite entsprechend der zur Verfügung stehenden Höhe Niederhalter eintauchen. Die mittels der erfindungsgemäßen Vorrichtung erzeugten gewellten Bahnen können über die gesamte Bahnbreite dieselbe oder bei entsprechender Gestaltung des Führungsbetts auch unterschiedliche Wellenhöhe aufweisen. Es versteht sich für den Fachmann von selbst, daß das Führungsbett auch nur eine Rille aufweisen kann, wenn die gewünschte gewellte Bahn entsprechende Eigenschaften aufweisen soll. Dann ist auch nur ein Niederhalter notwendig. Die Niederhalter sind vorzugsweise stabförmig ausgebildet und vorzugsweise sowohl an dem dem Eintrittsende des Führungsbetts als auch an dem dem Austrittsende des Führungsbetts zugewandten Ende senkrecht zum Führungsbett frei verschiebbar. Die Niederhalter können an beiden Enden gehaltert sein oder ein freies, nicht geführtes oder gelagertes Ende aufweisen. Es ist von Vorteil, wenn die Niederhalter eine gewisse Elastizität aufweisen. Federstahl ist ein geeignetes Material für die Niederhalter. Die Niederhalter können in zumindest einer senkrecht zum Führungsbett verlaufenden Führungsfläche geführt sein. Drücken die Niederhalter zusätzlich durch kraftausübende Mittel mit einer vorgegebenen Kraft die Bahn gegen das Führungsbett, so wird der laufenden Bahn ein gewisser Einstelleffekt ermöglicht, welcher Spannungsunterschiede im Material durch die nicht immer geometrisch exakte Faltenbildung ausgleicht. Die vorgegebene Kraft auf die Niederhalter kann auch durch eine Vorspannung der Niederhalter ausgeübt werden. Zusäztliche kraftausübende Elemente können dann entfallen. Neben einem runden Querschnitt, der besonders bevorzugt ist, können die Niederhalter beispielsweise auch einen dreieckigen, rechteckigen, halbrunden oder trapezförmigen Querschnitt aufweisen.

Besonders günstig ist es, wenn die freien Enden der Niederhalter im wesentlichen parallel zueinander abgebogen oder mit entsprechenden Ansätzen versehen sind, die in Bohrungen von zumindest einem endseitig gelegenem Führungselement aufgenommen werden. Es ist des weiteren von Vorteil, wenn alle Niederhalter bezüglich der Laufrichtung der Bahn vor bzw. nach dem Oberflächenbereich des Führungsbetts, welches die Rillen aufweist, aufgenommen sind. Die Niederhalter weisen vorzugsweise einen runden Querschnitt auf, wobei der Durchmesser beispielsweise von 0,1 bis 5 mm, vorzugsweise 1 bis 3 mm, insbesondere 2 mm, betragen kann. Der Abstand der Rillen kann z.B. 1 bis 5 10 mm, vorzugsweise 2 bis 4 mm, betragen.

Die Mittel zur Kraftausübung können beispielsweise eine Druckplatte sein, welche zwischen den Enden der Niederhalter auf diese einwirkt. Die Druckplatte kann zusätzlich mit zumindest einem Federelement versehen sein. Auch Gewichte, Druckluft, Vakuum oder Magnete können zur Kraftausübung eingesetzt werden. Werden Magnete verwendet, sind sowohl Permanent- als auch Elektromagnete geeignet, welche auf der der zu bildenden Bahn gegenüberliegenden Seite des Führungsbetts angeordnet sein sollten. Daß bei der Verwendung von Magenten die Niederhalter oder ein auf die Niederhalter einwirkendes Mittel, beispielsweise eine aufliegende Druckplatte, ferromagnetische Eigenschaften haben sollten, ist für den Fachmann klar.

Die mittels der erfindungsgemäßen Vorrichtung oder des erfindungsgemäßen Verfahrens erzeugten gewellten Bahnen aus dünnem Material müssen nach dem Austritt aus dem Führungsbett fixiert werden, damit die Wellenbildung dauerhaft bleibt. Eine Möglichkeit, die gewellte Bahn zu fixieren ist die Verbindung mit einem bahnförmigen Material (Trägerbahn) oder mehreren bahnförmigen Materialien (Trägerbahnen). Eine andere Möglichkeit der Fixierung besteht darin, die gewellte Bahn zwischen Prägewalzen dauerhaft plastisch zu verformen. Es ist weiterhin möglich, die gewellte Bahn durch Aufsprühen eines Verfestigungsmittels in der Wellenform zu fixieren. Wird eine Trägerbahn zur Stabilisierung der gewellten Bahn verwendet, so erfolgt die Verbindung zwischen den Bahnen vorzugsweise durch ein Haftmittel wie einen Klebstoff. Die Verbindung kann aber beispielsweise auch durch thermische Einwirkung oder Ultraschallschweißung erfolgen.

Gemäß einer weiteren Ausführungsform ist das Führungsbett im Querschnitt mit unterschiedlicher Wellenhöhe ausgebildet, wobei in der Regel in der Mitte eine maximale Wellenhöhe geformt wird, welche zu den Rändern des Führungsbetts hin abnimmt.

Es ist des weiteren von Vorteil, wenn das Führungsbett, quer zur Laufrichtung der Materialbahn gesehen, so ausgestaltet ist, daß die Rillen nicht alle auf gleicher Höhe beginnen. Ein Beginn der Rillen im Mittelbereich des Führungsbetts ist dabei besonders bevorzugt, wobei dann weiter stromabwärts beginnende Rillen in den Randbereichen angeordnet sind. Auch ist es für manche Ausführungsformen wünschenswert, wenn das Führungsbett so ausgebildet ist, daß nicht die gesamte Breite mit Rillen versehen ist, sondern im Mittelteil und/oder in Randbereichen des Führungsbetts keine Rillen vorgesehen sind.

Schließlich kann die erfindungsgemäße Vorrichtung eine Einrichtung mit kämmenden Walzen zur Prägung der erzeugten gewellten Bahn aufweisen, wobei die Einrichtung mit kämmenden Walzen dem Führungsbett nachgeschaltet ist.

Die mit dem erfindungsgemäßen Verfahren und der erfindungsgemäßen Vorrichtung hergestellte gewellte Bahn kann vorteilhafterweise als Bestandteil eines absorbierenden Artikels zur Aufnahme von Körperflüssigkeiten, wie beispielsweise einer Windel, einer Damenbinde oder einer Inkontinenzeinlage, eingesetzt werden. Ein entsprechender absorbierender Artikel weist üblicherweise eine bei Gebrauch dem Körper abgewandte flüssigkeitsundurchlässige Abdecklage, eine bei Gebrauch dem Körper zugewandte flüssigkeitsdurchlässige Abdecklage und einen zwischen der flüssigkeitsdurchlässigen und der flüssigkeitsundurchlässigen Abdecklage angeordneten Saugkörper auf. Der erfindungsgemäße absorbierende Artikel ist dadurch ausgezeichnet, daß die flüssigkeitsdurchlässige Abdecklage und/oder der Saugkörper zumindest in Teilbereichen eine gewellte ("plissierte") Form aufweisen, wobei Bahnen verwendet werden können, die mittels dem erfindungsgemäßen Verfahren oder der erfindungsgemäßen Vorrichtung in Falten gelegt wurden. Gegenüber wie herkömmlich mittels Prägewalzen hergestellten gewellten Bahnen haben die erfindungsgemäß hergestellten Bahnen den Vorteil eines angenehmen Tragekomforts bzw. einer verbesserten Saugleistung, da das bearbeitete Material beim Formungsvorgang praktisch nicht verfestigt wird.

Die Erfindung wird nachfolgend anhand der Zeichnungen näher erläutert. Es zeigen
- Fig. 1: eine perspektivische, teilweise aufgeschnittene Ansicht einer Maschine zur Herstellung einer gewellten Bahn aus sehr elastischem Material, die mit einer Trägerbahn verbunden wird,
- Fig. 2: eine perspektivische Draufsicht auf die gewellte Bahn, die zugleich der Form des Führungsbetts in einer bevorzugten Ausführung darstellt,
- Fig. 3: eine perspektivische Darstellung einer alternativen, in der Mitte beginnenden Wellenausbildung,
- Fig. 4: eine perspektivische Draufsicht auf eine Bahn bzw. ein Führungsbett mit nur teilweiser Wellenbildung,
- Fig. 5: einen Querschnitt durch drei Sektionen des Führungsbetts am Anfang, in der Mitte und am Ende mit einer schematischen Darstellung der eingezogenen Bahn,
- Fig. 6: verschiedene theoretische Ausformmöglichkeiten der gewellten Bahn mit unterschiedlichen Einzugsfaktoren,
- Fig. 7: materialtechnische Betrachtungen für vorzugsweise zu verarbeitende Bahnen,
- Fig. 8: eine Seitenansicht des Führungsbettendes mit einer Verbindungseinrichtung sowie die Fixierung einer Andruckmöglichkeit der Niederhalter mittels Pneumatikzylinder und Kniehebel,
- Fig. 9: eine teilweise aufgeschnittene perspektivische Ansicht des Führungsbetts und der Niederhalter, auf welche hier durch getrennt ansteuerbare Elektromagnete jeweils abschnittsweise veränderliche Kräfte aufgebracht werden können,
- Fig. 10: eine Ansicht der beiden Walzen der Verbindungsstation mit den in die gewellte Walze eingreifenden Niederhaltern und streifenförmigem Leimauftrag,
- Fig. 11: eine schematische Darstellung von nachgeschalteten Prägewalzen, welche die gewellte Bahn an den Wellenspitzen zur Fixierung durch Pressen verformen,
- Fig. 12: eine schematische Darstellung der Aufbringung einer zweiten Bahn mit der anschließenden Preßstation zur Fixierung der Klebeverbindung,
- Fig. 13: eine schematische Darstellung einer weiteren bevorzugten Ausführung zur Verklebung der zweiten Bahn, und
- Fig. 14 bis 21: schematische Darstellungen von absorbierenden Artikeln gemäß der Erfindung.

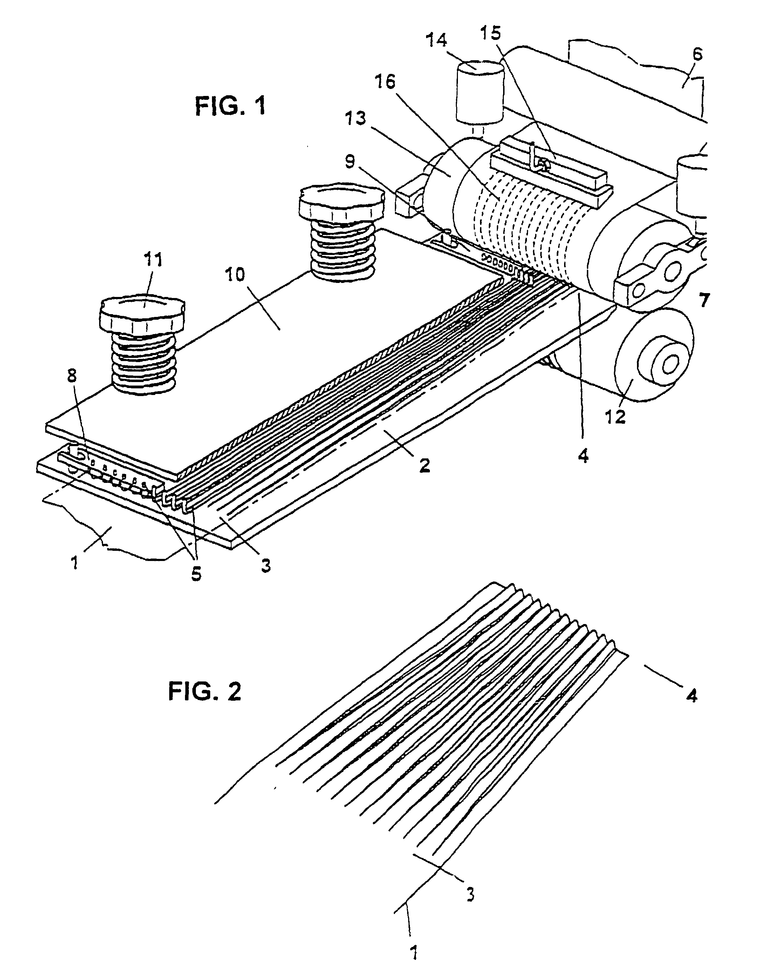

Die zu wellende Bahn 1 tritt gemäß Fig. 1 an der Vorderkante 3 des Führungsbetts 2 in die Wellvorrichtung ein, wird durch die stabförmig ausgebildeten Niederhalter 5 in das Führungsbett gedrückt und verläßt dies am Austrittsende 4 in gewellter Form. Die Niederhalter sind in einem vorderen Abstandshalter 8 und einem hinteren Abstandshalter 9 zusammengefaßt, so daß ein gegenseitiger Abstand konstant gehalten wird und sie werden bei der in Fig. 1 gezeigten Ausführungsform von einer über Einstellschrauben 11 mittels Federn beaufschlagten Druckplatte 10 mit einer definierten Kraft angedrückt. In Richtung auf das Führungsbett sind die Niederhalter frei verschiebbar gelagert.

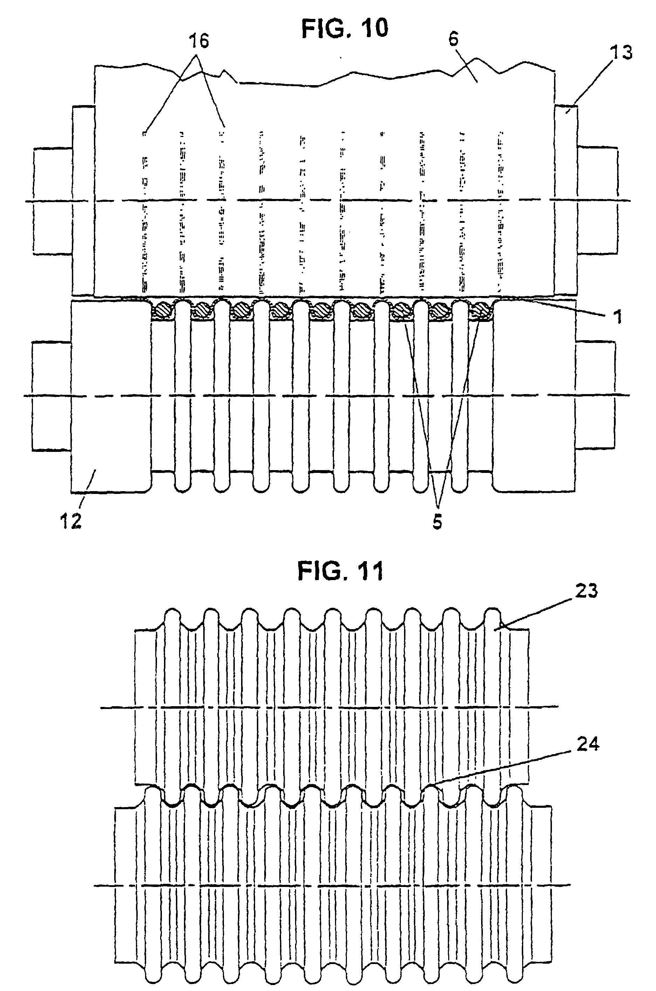

Unmittelbar nach dem Verlassen der Führungsbahn 2 erfolgt das Zusammenführen der gewellten Bahn mit der Trägerbahn 6, das über eine glatte Walze 13 und durch eine im Querschnitt profillierte Walze 12, wie auch in Fig. 10 als Ansicht dargestellt, bewirkt wird. Die für die anschließende Verklebung der Schichten erforderliche Anpreßkraft wird hier mittels zweier Pneumatikzylinder über Hebel aufgebracht.

Zum Verbinden der Trägerbahn mit der gewellten Bahn ist in diesem Falle eine Lösung mit linienförmigen Klebstoffauftrag 16 mittels einer Mehrfach-Leimauftragsdüse 15 dargestellt. Durch die besondere Form und Befestigung der stabförmigen Niederhalter, deren Enden bis in die profillierte Walze 12 reichen, was auch aus Fig. 10 ersichtlich ist, wird ein Zurückfedern der ausgeformten Wellenbahn vor dem Verkleben verhindert.

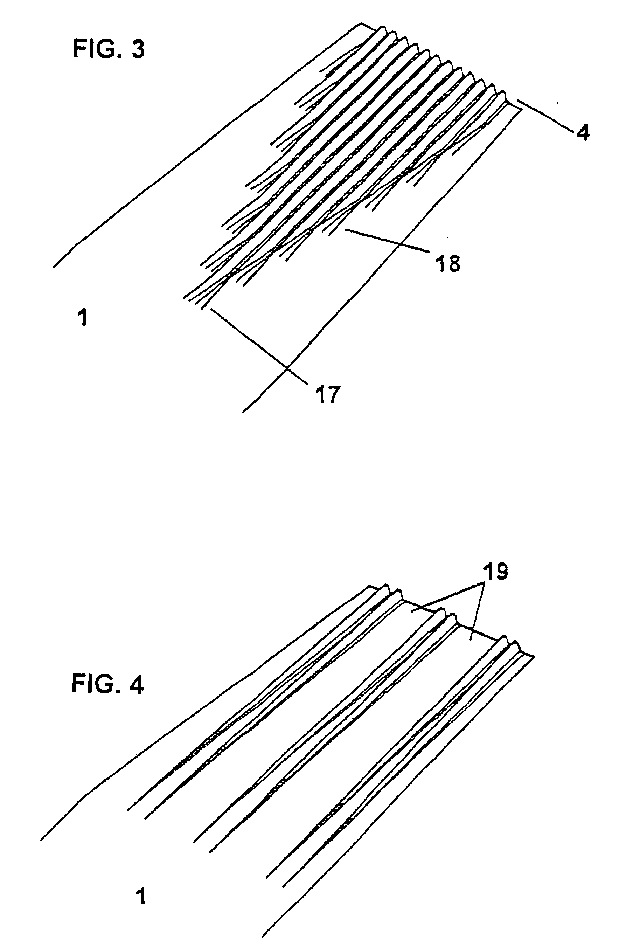

Die gewellte Bahn 3 in Fig. 2 entspricht im wesentlichen dem ebenen Führungsbett, man sieht ein geradliniges Ansteigen der Wellen, welches eine einfache Herstellung der Ausformeinrichtung ermöglicht. Um bei weniger dehnbarem Material die lokalen Spannungen zu verringern, kann das Führungsbett vorteilhaft entsprechend Fig. 3 ausgebildet werden, wo die Wellenbildung in der Mitte der Bahn beginnt und weiter am Rand liegende Wellen erst später ausgeformt werden. Allerdings ist bei einer solchen Ausführung der Einbau der Niederhalter mit erhöhtem Aufwand verbunden, da diese auf unterschiedlich langen Strecken die volle Wellenhöhe ausformen müssen und verschiedene Neigung gegen das Führungsbett aufweisen.

Für Bahnen, die entsprechend ihrer Anwendung nicht über die ganze Breite gewellt sein sollen, läßt sich das Führungsbett gemäß Fig. 4 auch für nur wenige Wellen mit dazwischenliegenden ebenen Führungsbettabschnitten 19 ausbilden. Im Extremfall kann das Führungsbett auch nur eine Rille aufweisen, wie vorstehend bereits angedeutet wurde.

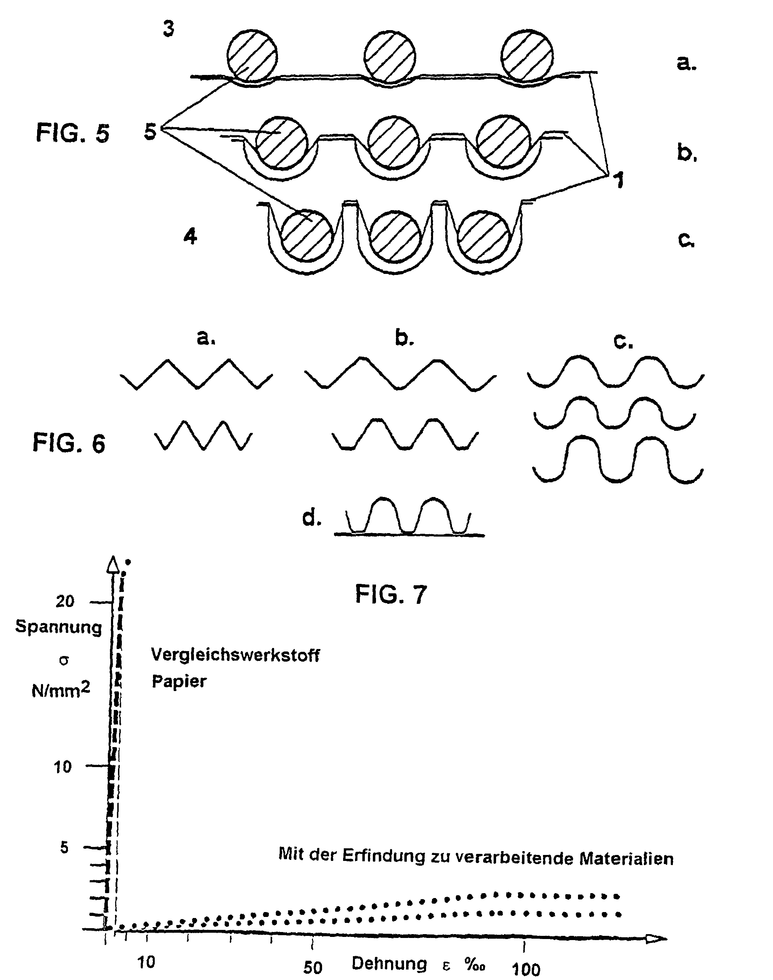

Die Ausgestaltung von Führungsbett und Niederhaltern kann zu einem nicht über die gesamte Länge der Wellenbildungsstrecke gleichmäßigen Einzugsverhalten führen, was in Fig. 5 schematisch dargestellt ist. Mittels eines Berechnungsprogramms kann der Einzugsfaktor für die zu wellende Bahn an jeder Stelle des Führungsbettes ermittelt und optimiert werden. Bei der erfindungsgemäßen Lösung wird am Beginn der Faltenbildung, dargestellt unter a, und am Ende gemäß Bild c, der ideale Wert erreicht, während etwa in der Mitte des Führungsbetts entsprechend Bild b nur ca. 92 Prozent des Materials eingezogen werden, was jedoch durch die Dehnbarkeit und geringe Biegesteife der Bahn ausgeglichen wird.

Je nach Aufgabengebiet sind die unterschiedlichsten Wellenformen gemäß Fig. 6 theoretisch herstellbar, jedoch würden die eckig ausgeformten Wellen nach a und b eine chemische oder physikalische Behandlung, beispielsweise durch Einsprühen mit einem Verfestigungsmittel, thermoplastische Verfestigung oder sehr starkes plastisches Ausprägen erfordern, um den gezeigten Querschnitt beizubehalten. Die Einzugswerte schwanken zwischen 1,41 bei 45 Grad Dreieckswelle bzw. 1,32 für die Trapezform mit einem Drittel Geradenabschnitt und dem Wert 2 bei 60 Grad Steigung der Welle bzw. 1,66 beim Trapez.

Für die bevorzugte Ausführungsform c mit Halbkreisen und mehr oder weniger langen Geradenabschnitten beträgt der Materialeinzug zwischen 1,45 bei kurzen Geradenstücken mit 50 Grad Neigung und Werten größer als 2 bei fast senkrechten Geradenanteilen. Eine Sonderstellung nehmen die beiden aneinandergereihten Halbkreise ein, hier beträgt der Einzugsfaktor genau π/2, also 1,57. Diese Wellenform ist auch von der Ausgewogenheit der inneren Spannungen und der eBelastbarkeit durch äußere Kräfte ohne zusätzliche, stabilisierende Maßnahmen vorteilhaft. Bei Verklebung mit einer Trägerbahn wird sich zwangsweise - je nach Verbindungsverfahren - eine gewisse Abplattung der Wellen einstellen, so daß der praktisch erzeugte Wellenquerschnitt die Form d annimmt.

Um die erfindungsgemäße Maschine durchlaufen zu können, sollte die zu verarbeitende Bahn bestimmte physikalische Eigenschaften aufweisen: geringe Biegesteifigkeit und hohe Dehnbarkeit. Diese Forderung wird beispielsweise von faserigen Stoffen geringer Dichte und Kunststoff-Folien erfüllt. In Fig. 7 ist eine Spannungs-Dehnungs-Kurve für verschiedene Materialien angegeben. Im Gegensatz zu Papier, bei dessen Herstellung auf möglichst gerine Deformierbarkeit und hohe Festigkeit Wert gelegt wird, besitzen solche Stoffe eine gewisse Verformbarkeit, verbunden mit einer meist nicht genau definierten Streckgrenze und hoher Bruchdehnung. Vorzugsweise sind die verarbeiteten Materialbahnen zumindest in Querrichtung elastisch.

Im Falle der Wellenausbildung wird gegen Ende des Ausformvorgangs rechnerisch von der Bahn in Querrichtung eine etwa 8 % längere Strecke als in der Mitte des Vorgangs gefordert, was jeder der bisher untersuchten Stoffe problemlos überstand, da dies allenfalls zu einer minimalen, bleibenden Deformation führt, die im vorliegenden Anwendungsfall noch nicht einmal unerwünscht ist. Folglich ist die Maschine mit dem einfach ausgebildeten Führungsbett zur Wellenbildung in dünnen Bahnen insbesondere für alle Werkstoffe, welche ca. 10 % Dehnung und mehr ohne Bruch erreichen, besonders geeignet. Obwohl keine Obergrenze für die Dehnung besteht, sind Materialien bevorzugt, welche eine Dehnung von 10 bis 150 % zulassen.

In der nachfolgend angegebenen Tabelle 1 sind einige Beispiele von Materialien bzw. Materialkombinationen angegeben, welche mit der erfindungsgemäßen Vorrichtung und mittels des erfindungsgemäßen Verfahrens zur Herstellung gewellter Bahnen verarbeitet werden können. Die hergestellten gewellten Bahnen werden auch als "plissierte" Bahnen bezeichnet und das dazu verwendete Material entsprechend als "plissierbares" Material.

**Tabelle 1**

| **Materialien** | **Dimensionen** | **Kardenvlies** | **Spinnvlies** | **Laminat Kardenvlies/ Lochfolie** | **Laminat Spunbond/ Lochfolie** |
|---|---|---|---|---|---|
| **Flächengewicht** | **g/m**^{**2**} | **18** | **16** | **43** | **42** |
| **Dicke** | **mm** | **0,18** | **0,13** | **0,92** | **0,50** |
| **Festigkeiten, trocken** | | | | | |
| **längs** | **N/50 mm** | **40** | **30** | **55** | **13** |
| **quer** | **N/50 mm** | **18** | **20** | **19** | **6** |
| **Dehnung, trocken** | | | | | |
| **längs** | **%** | **50** | **50** | **35** | **34** |
| **quer** | **%** | **90** | **60** | **150** | **78** |
| **Reibung, trocken** | | | | | |
| **Innen(Folien)-seite** | **µD** | **0,65** | **0,33** | **0,41** | **0,30** |
| **Außen(Vlies)-seite** | **µD** | **0,65** | **0,33** | **0,41** | **0,93** |

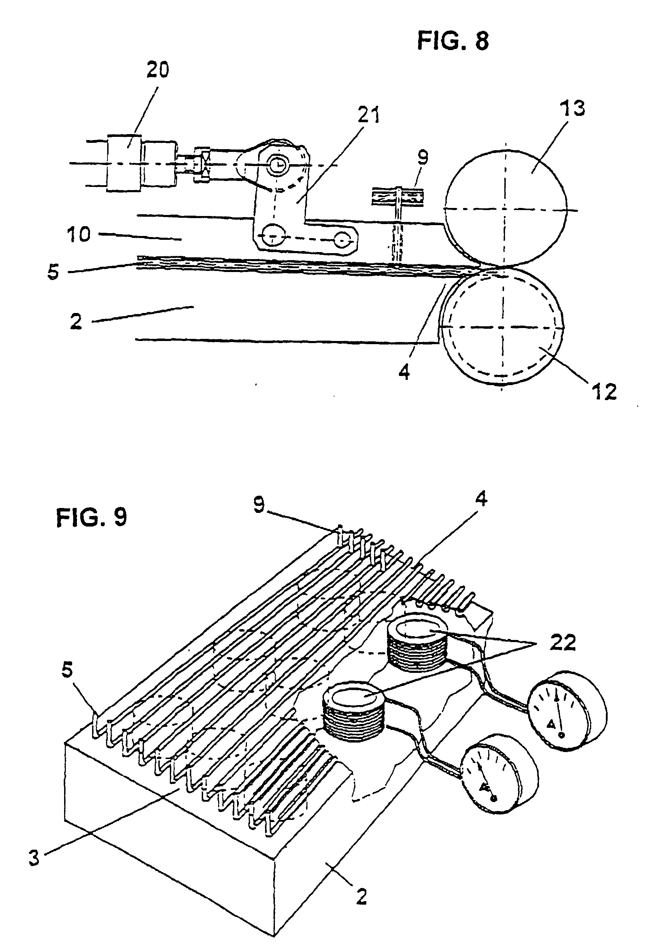

Günstig für die gleichmäßige Wellenausbildung mit der erfindungsgemäßen Vorrichtung ist eine definierte Druckkraft auf die Bahn, welche durch die Niederhalter aufgebracht wird. Diese kann durch Gewichte, Federkraft (siehe Fig. 1) oder auch während des Betriebs steuerbar entsprechend Fig. 8 über Pneumatik- oder Hydraulikzylinder 20 geschehen, wobei wegen der geringen erforderlichen Kräfte und weniger Leckage einem Pneumatikzylinder der Vorzug zu geben ist. Zur Einsparung von Bauhöhe kann dieser z.B. längs angeordnet werden und über Kniehebel 21 auf die Andrückplatte 10 wirken. Nach theoretischen Untersuchungen und praktischen Versuchsergebnissen hat sich je nach verarbeitetem Material eine Linienlast von 0,04 bis 0,06 Ncm⁻¹ für Bahngeschwindigkeiten bis 150 m/min günstig erwiesen. Die Gesamtauflagekraft erreicht damit bei einer Länge des Führungsbetts von 300 mm etwa 20 N. Bei Veränderung des Flächengewichts und/oder der Biegesteifigkeit des Materials ist auch eine Kraft von beispielsweise 0,01 bis 0,1 Ncm⁻¹, vorzugsweise 0,02 bis 0,08 Ncm⁻¹ geeignet. Die Lösung mit Pneumatikzylinder hat neben der Steuerbarkeit der Niederhalterkräfte auch den Vorteil, daß zum Einfädeln der Bahn die Einrichtung pneumatisch angehoben und entlastet werden kann. Mit geeigneten Materialien können problemlos Bahngeschwindigkeiten von bis zu 250 m/min erzielt werden.

Fig. 8 zeigt eine Seitenansicht der bis in die profillierte Walze 12 reichenden Niederhalterstäbe 5 mit der über Klemmschrauben einstellbaren hinteren Halterung 9.

Um eine noch individuellere Kraftverteilung in den Niederhaltern zu erzielen, können in das Führungsbett gemäß der in Fig. 9 gezeigten bevorzugten Ausführungsform der erfindungsgemäßen Vorrichtung Elektromagnete 22 eingelassen werden, die je nach Erfordernissen gruppenweise oder einzeln zu beaufschlagen sind, wodurch sich eine besonders feinfühlige Einflußnahme auf die Wellenausbildung ergibt. Die ferromagnetischen Niederhalterstäbe 5 werden von Elektromagneten mit bis zu 0,1 Nmm⁻² angezogen, d.h. bei einer Stabbreite von 2 mm genügt entweder ein sehr geringer Strom oder eine entsprechend verteilte geringe Anzahl von Einzelmagneten, um den erforderlichen Anpreßdruck zu erzeugen. Diese Lösung hat den Vorteil, daß trotz eines eventuell unterschiedlichen Verschleißes der Niederhalterstäbe dennoch eine gleichmäßige Wellenausbildung möglich ist, ohne daß Nachstellarbeiten erforderlich werden.

Wegen der Instabilität des verarbeiteten Materials würden die ausgeformten Wellen ohne zusätzliche Vorkehrungen wie etwa einer Verbindung mit einer Trägerbahn, einer thermoplastischen Veränderung oder einem Fixieren mittels eines aufgesprühten Verfestigungsmittel nach Verlassen der Formeinrichtung in den ebenen Zustand zurückgefedert.

Wenn eine Trägerbahn zur Stabilisierung verwendet wird, kann diese dabei aus dem gleichen Material wie die gewellte Bahn oder auch aus einem anderen Material bestehen. Die Verbindung mit einer Trägerbahn erfolgt vorzugsweise, wie in den Fig. 1 und 10 gezeigt, durch Verkleben einer glatten Bahn 6 mit den Spitzen (Maxima bzw. Minima) der ausgeformten Wellenbahn 1. Die Wellen werden während dieses Vorgangs in einer entsprechend dem Abstand der Niederhalter 5 mit Nuten versehenen Walze 12 und den Stabenden der Niederhalter zwangsweise gehalten, während eine von oben z.B. über Pneumatikzylinder angedrückte Walze die zweite Bahn 6 zuführt, auf die mittels einer Mehrfachdüse jeweils im Bereich der Wellenberge linienförmig Klebstoff 16 aufgebracht wird.

Das Verbinden von gewellter Bahn mit Trägerbahn gemäß Fig. 1 durch linienförmigen Klebstoffauftrag 16 ist nur eine der hierfür geeigneten Möglichkeiten. Auch der Einsatz von Rad- bzw. Siebdruck-Leimauftragsgeräten ist denkbar.

Da die erfindungsgemäße Vorrichtung vorzugsweise zur Verarbeitung von Bahnen aus synthetischen Folien oder Vliesen eingesetzt werden kann, kann durch Einbringen thermischer Energie beispielsweise über die gewellte Transportwalze 12 ein Verschweißen der gebildeten Wellenbahn mit der Trägerbahn erfolgen.

Einen ähnlichen Verbindungseffekt bewirkt das Zusammendrücken der Bahnen im Wellental der aufliegenen gewellten Bahn z.B. durch einen mit Ultraschall beaufschlagten Preßschuh oder eine Preßwalze.

Um die ausgebildete Wellenform auch ohne zusätzliche Trägerbahn zu fixieren, kann die gewellte Bahn vorzugusweise gemäß Fig. 11 zwischen Prägewalzen 23 mit wellenförmigem Querschnitt in den Wellenspitzen 24 plastisch verformt werden. Dieser Effekt kann außerdem durch thermische oder chemisch-pyhsikalische Einwirkung, wie etwa dem Aufsprühen eines Verfestigungsmittels, erreicht oder verstärkt werden.

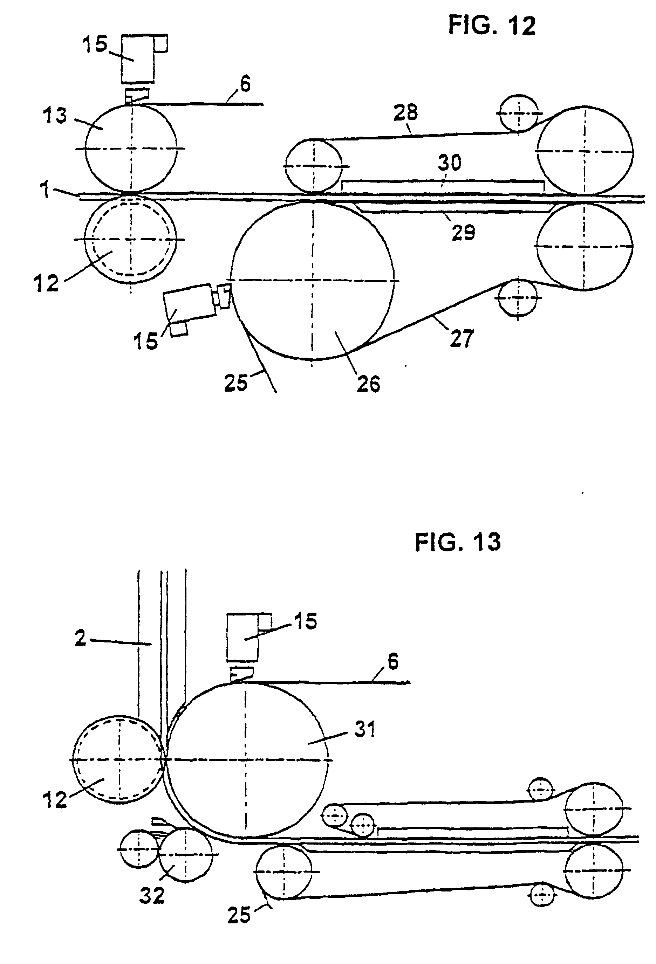

Eine weitere vorteilhafte Ausführung der Erfindung besteht gemäß Fig. 12 darin, die der Trägerbahn 6 gegenüberliegende Seite der Wellenbahn ebenfalls mit einer weiteren Materialbahn 25 zu verbinden. Die Materialbahn 25 wird auch als Deckbahn bezeichnet. Zu diesem Zweck kann beispielsweise diese Bahn mittels einer Walze größeren Durchmessers 26 und einem Riemensystem 27 zugeführt werden, wobei mittels einer Auftragsvorrichtung 15 streifenförmig Klebemittel aufgebracht wird. In einer vorteilhaften Ausführung kann durch ein zweites Riemensystem 28 und eine Andrückvorrichtung 30, welche den Anpreßdruck gleichmäßig verteilt, die Verklebung mit der Wellenbahn 1 erfolgen, wobei die Unterstützung 29 dem Anpreßdruck entgegenwirkt.

Gemäß Fig. 13 wird die aus dem Führungsbett 2 austretende Wellenbahn mittels einer gewellten Walze 12 gegen eine glatte Walze 31 größeren Durchmessers gedrückt, wobei sie mit der Trägerbahn 6, auf die beispielsweise vorher mittels einer geeigneten Vorrichtung 15 streifenförmig Klebstoff aufgebracht wurde, verbunden wird.

Die Krümmung der Walze 31 ist so gewählt, daß die Wellenbahn lediglich geringfügig elastisch verformt wird und nach dem Verlassen der Walze ihren ursprünglichen Querschnitt wieder einnimmt. Mittels eines im Prinzip bekannten Leimwerks 32 kann in einem Bereich der Walze 31 auf die Wellenspitzen Klebstoff aufgetragen werden. Durch eine anschließende Anpreßstrecke mit kontrolliertem Anpreßdruck, wie sie unter Fig. 12 beschreiben ist, wird die Verbindung gefestigt.

Die Erfindung betrifft des weiteren einen absorbierenden Artikel, welcher zur Aufnahme von Körperflüssigkeiten geeignet ist. Derartige Artikel sind beispielsweise Artikel für die Frauenhygiene, wie Damenbinden, sowie Windeln, Inkontinenzeinlagen oder dergleichen.

Die vorgenannten Hygieneartikel sind in verschiedensten Ausführungsformen bekannt. Diesen ist eine rückseitige, flüssigkeitsdurchlässige Abdecklage, eine vorderseitige, flüssigkeitsdurchlässige Abdecklage und ein zwischen diesen beiden Lagen angeordneter Saugkörper gemeinsam.

Die rückseitige Abdecklage ist üblicherweise aus einer dünnen Polyethylen-Folie gebildet. Für die vorderseitige, flüssigkeitsdurchlässige Abdecklage werden meist Vliesstoffe eingesetzt. Auch die Verwendung gelochter Folien ist bekannt.

Der Saugkörper besteht in der Regel aus Zellstoff-Flocken oder einer luftgelegtten Faserbahn - einem sogenannten Air-Laid-Material.

Neben der grundsätzlichen Saugleistung des Saugkörpers bilden produkttechnische Eigenschaften der vorderseitigen - also dem Körper des Trägers zugewandten - Abdecklage, wie taktile Weichheit, Ansaugzeit bezüglich der Körperflüssigkeit, deren Verteilung über den Hygieneartikel und die Rücknäßeigenschaften eine entscheidende Rolle. Die üblicherweise für die körperseitige Abdecklage verwendeten Vliesstoffe sind in ihren diesbezüglichen Eigenschaften weitgehend vergleichbar.

Durch die erfindungsgemäße Faltenlegung der vorderseitigen Abdecklage wird ein Oberflächeneffekt erzielt, der am ehesten mit dem Ausdruck "Plissierung" umschrieben werden kann.

Durch die Falten werden gleichzeitig Längskanäle an der Oberfläche des Hygieneartikels zur Verbesserung der Flüssigkeitsverteilung in Längsrichtung und Barrieren gegen eine Flüssigkeitsausbreitung in Querrichtung geschaffen. Dies verbessert erheblich die Auslaufsicherheit des Hygieneartikels.

Weiterhin wird durch die Falten in erheblichen Bereichen der Abdecklage ein Abstand zwischen diesen und dem darunter befindlichen Saugkörper erzielt, der das Ansaug- bzw. Eindringverhalten der Körperflüssigkeit in den Saugkörper und gleichzeitig die Rücknäßeigenschaften verbessert. Auch wird der visuelle Eindruck nach einer Beaufschlagung z.B. durch Menstruationsflüssigkeit, verbessert, da die erhabenen Faltenbereiche nicht mit dem flüssigkeitsgetränkten Saugkörper in Verbindung stehen. Nicht zuletzt wird durch die Nachgiebigkeit der Falten die Weichheit der dem Körper zugewandten Oberfläche des Hygieneartikels beeinflußt, was den Tragekomfort und den natürlichen Griff verbessert.

Durch die Ausbildung eines Teils des Saugkörpers in plissierter Form kann ein entsprechender Effekt hinsichtlich einer Verbesserung der Flüssigkeitsverteilung ebenfalls erreicht oder in Kombination mit einer plissierten Abdecklage sogar noch verbessert werden. Geeignete Materialien für den plissierten Saugkörper sind hydrophile Vliese, wie hydrophile Kardenvliese oder hydrophile Spinnvliese mit Flächengewichten von 6 bis 80 g/m², insbesondere 10 bis 30 g/m², luftgelegte Faserbahnen und Laminate.

Für die plissierte Abdecklage können hydrophobe Vliese eingesetzt werden, wie hydrophobe Kardenvliese und hydrophobe Spinnvliese. Das Flächengewicht entsprechender Materialien liegt wiederum vorzugsweise im Bereich von 5 6 bis 30 80 g/m², insbesondere 10 bis 20 g/m².

Wenn gemäß einer bevorzugten Ausführungsform die dem Saugkörper zugewandten Fußbereiche der Falten auf dem Saugkörper oder einer zwischen diesem und der vorderseitigen Abdecklage angeordenten Trägerlage fixiert sind, werden die Falten so stabilisiert, daß die von ihnen hervorgerufenen positiven Effekte auch nach einer Lagerung im gepreßten Zustand und über eine längere Tragezeit erhalten bleiben.

Die Fußbereiche der Falten können durch eine Verklebung (Klebstoff-Streifen) oder Verschweißung auf dem Saugkörper oder der Trägerlage auf einer Breite (Fußbreite F) von vorzugsweise 0,2 bis 10 mm fixiert werden.

Die Trägerlage kann dabei die funktionellen Eigenschaften der Falten etwa hinsichtlich Flüssigkeitsweiterleitung und Rücknässung verbessern.

Die Fußbereiche zweier benachbarter Falten können mit einem gegenseitigen Abstand (A) von 1 bis 20 mm fixiert sein und die Abstehhöhe (II) der Falten in unbelastetem Zustand kann ebenfalls 1 bis 20 mm betragen.

Gemäß einer bevorzugten Ausführungsform können mehrere Falten über die gesamte Breite der Saugkörperoberfläche verteilt vorgesehen sein. Damit wird eine maximale Produktverbesserung erzielt. 5 bis 25 Falten über die gesamte Breite haben sich als besonders günstig erwiesen. Schließlich können mehrere, vorzugsweise 2 bis 10 Falten jeweils in auf eine Teilbreite der Saugkörperoberfläche begrenzten, randseitigen und/oder mittigen Streifen vorgesehen sein.

Der erfindungsgemäße absorbierende Artikel wird des weiteren anhand der beigefügten Zeichnung näher erläutert.

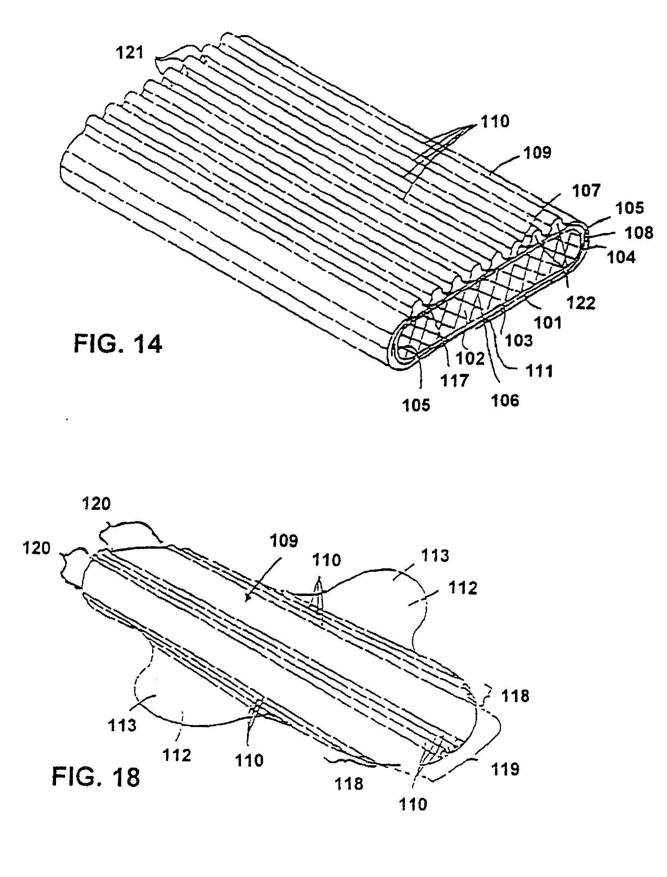

Wie aus den beigefügten Figuren 14 bis 20 deutlich wird, weisen die verschiedenen Ausführungsformen der gezeigten Damenbinde einen in wesentlichen Punkten übereinstimmenden Grundaufbau auf. So ist als rückseitige, flüssigkeitsundurchlässige Abdecklage eine Wäscheschutzfolie 101 vorgesehen, die dem als Zellstoff-Flockenkissen ausgebildeten Saugkörper 102 im Bereich seiner Rückseite 103 - also der der Trägerin im angelegten Zustand der Damenbinde körperabgewandten Seite - unterliegt. Die Wäscheschutzfolie 101 greift ferner mit ihren Längskanten 104 um den Seitenrand 105 des Saugkörpers 102 herum.

Auf der vorderseitigen Oberfläche 106 verläuft eine flächig aufgelegte Trägerfläche 107, bei der es sich beispielsweise um eine vliesstofflage handeln kann. Auch die Trägerlage 107 greift mit ihren Längskanten 108 um den Seitenrand 105 des Saugkörpers 102 herum und überlappt mit den Längskanten 104 der Wäscheschutzfolie 101. Im Überlappungsbereich sind diese beiden Teile in üblicher Weise miteinander verklebt.

Auf der Trägerlage 107 ist eine vorderseitige, flüssigkeitsdurchlässige Abdecklage 109 vorgesehen, die in noch näher zu erläuternder Weise mit Falten 110 versehen ist. Bei den in Fig. 14, 15 und 17 gezeigten Ausführungsformen der Damenbinde handelt es sich um eine sogenannte "Volleinschlagbinde", bei der die Abdecklage 109, die beispielsweise aus einem Polypropylen-Kardenvlies gebildet ist, um den Saugkörper 102 die Trägerlage 107 und die Abdecklage 109 auf der Rückseite 103 herumgeschlagen und mittig durch eine Klebeverbindung 111 fixiert.

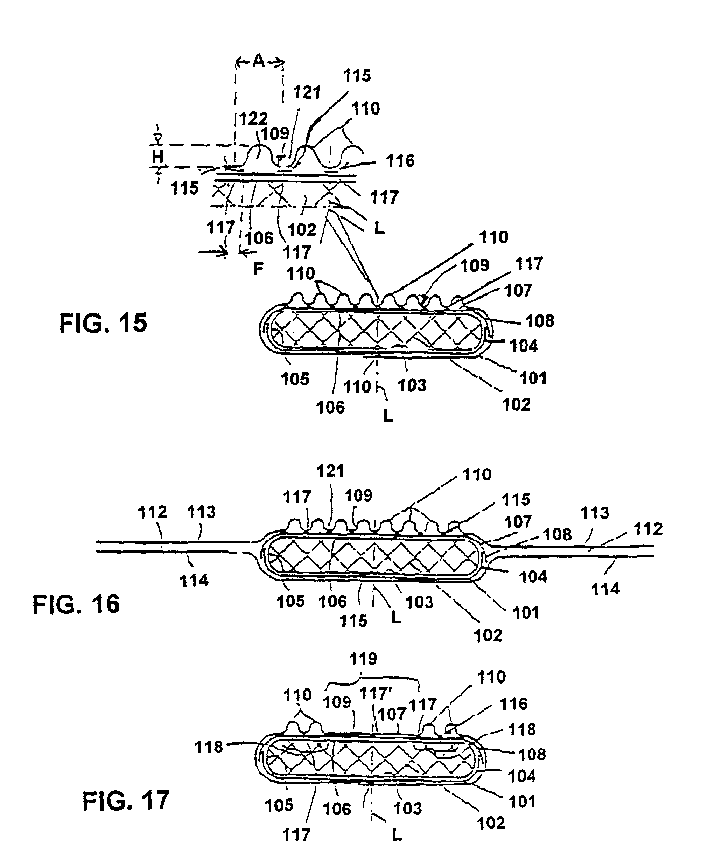

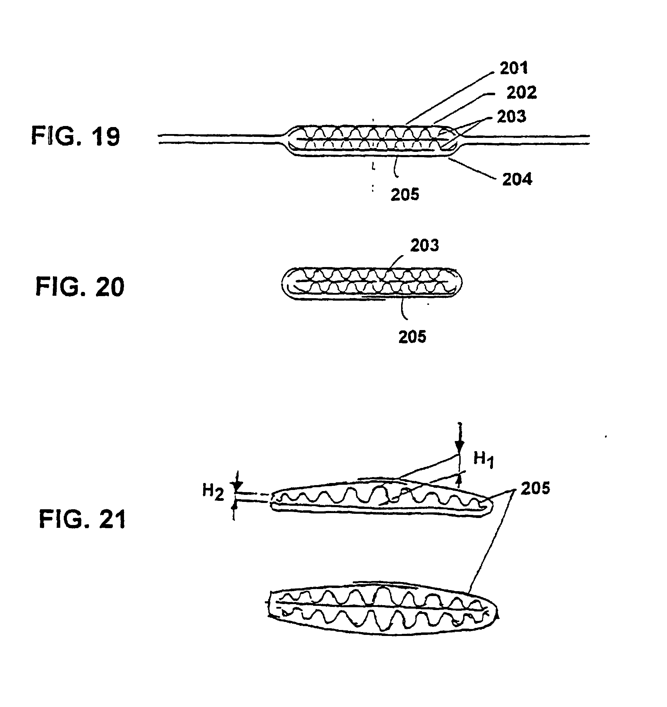

Bei den in Fig. 16 und 18 gezeigten Ausführungsformen sind seitliche Flügel 112 vorgesehen, die durch seitlich überstehende, miteinander randseitig verbundene Lappen 113, 114 der vorderseitigen Abdecklage 109 bzw. einer rückseitigen Decklage 115 gebildet sind. Die Decklage 115 kann aus Vliesstoff gebildet sein, wie dies insbesondere bei der in Fig. 16 gezeigten Ausführungsform der Fall ist, wo eine eigene Wäscheschutzfolie 101 vorgesehen ist. Die Decklage kann aber auch unter Weglassung der Wäscheschutzfolie 101 aus flüssigkeitsundurchlässigem Material, beispielsweise Polyethylen-Folie gefertigt sein, um somit die Wäscheschutzfunktion zu übernehmen.

Im folgenden werden die unterschiedlichen Faltenkonfigurationen, wie sie in den einzelnen Ausführungsformen der gezeigten Damenbinden verwendet werden, näher erläutert.

Die Falten 110 werden durch einen geeigneten Faltenleger in der Binden-Herstellungsmaschine so aufgebracht, daß die Fußbereiche 116 mit einem Abstand A in Querrichtung der Binde auf der Trägerlage 107 zu liegen kommen, der im Bereich zwischen 1 und 20 mm liegt. Als besonders praktikabel hat sich ein Abstandsbereich von 3 bis 5 mm herausgestellt. Die Fußbereiche 116 werden durch längsverlaufende Klebstoff-Streifen 117, die beispielsweise aus einem Sprühkleber gebildet sind, auf der Trägerlage 107 fixiert. Zwischen jeweils einander benachbarten Fußbereichen 116 steht die Falte 110 nach oben ab, wobei eine Abstehhöhe H im Bereich von 1 bis 20 mm denkbar ist. Als besonders praktikabel haben sich wiederum Werte für die Abstehhöhe herausgestellt, die im Bereich zwischen 3 und 5 mm liegen. Die durch die Klebstoff-Streifen 117 definierte Fußbreite F kann je nach Abstand A und Abstehhöhe H zwischen 0,2 und 10 mm betragen, wobei darauf zu achten ist, daß die Fußbreite F maximal die Hälfte des Abstands A beträgt. Bei einem Abstand A und einer Abstehhöhe H von 3 bis 5 mm haben sich Fußbreiten von 0,5 bis 1 mm bewährt.

Bei den in Fig. 14 bis 16 gezeigten Ausführungsbeispielen sind acht über die gesamte Breite der Saugkörperoberfläche 102 gleichmäßig verteilte Falten 110 vorgesehen, wobei mit einem Faltenabstand A von ca. 6 mm gearbeitet wird.

Bei der in Fig. 17 gezeigten Ausführungsform sind in zwei randseitigen Streifen 118 jeweils zwei in Längsrichtung der Damenbinde verlaufende Falten 110 vorgesehen, die wiederum einen Abstand A und eine Abstehhöhe H von ca. 6 mm aufweisen. In dem zwischen den beiden randseitigen Streifen 118 verbleibenden Mittenstreifen 119 liegt die flüssigkeitsdurchlässige Abdecklage 109 plan auf der Trägerlage 107 auf und ist durch einen mittigen Klebstoff-Streifen 117 zusätzlich it dieser verbunden. Auch eine vollflächige, flüssigkeitsdurchlässige Verklebung mit geringen Klebstoffmengen ist möglich.

Bei der in Fig. 18 skizzierten Ausführungsform einer Damenbinde ist zusätzlich zu den Falten 110 in den beiden randseitigen Streifen 118 symmentrisch zur Mittelebene L eine weitere Faltenkonfiguration mit drei Falten 110 vorgesehen, wobei faltenlose Streifen 120 zwischen den mittigen Falten 110 und den randseitigen Falten 110 verbleiben.

Wie aus den Querschnitten gemäß Fig. 15 bis 17 deutlich wird, bilden einerseits die auf der Außenseite der Falten 110 zwischen diesen liegenden Rinnen 121 und andererseits die unter den Falten 110 zwischen den verklebten Fußbereichen 116 liegenden Röhren 122 Kanäle zur Verteilung der die Damenbinde beaufschlagten Körperflüssigkeiten in Längsrichtung der Binde.

Es ist darauf hinzuweisen, daß für die einzelnen Komponenten der erfindungsgemäßen Binde jeweils geeignete Materialien aus dem zur Verfügung stehenden Angebot nach den üblichen Kriterien für die Konstruktion solcher Hygieneartikel auszusuchen und geeignete Konstruktionsmaßnahmen zu treffen sind. Zu letzteren wird nur beispielhaft darauf hingewiesen, daß die Klebstoff-Streifen 117 so ausgelegt sein sollten, daß sie die Oberfläche der Trägerlage 107 nur im möglichst geringen Maße gegen einen Flüssigkeitsdurchtritt verschließen, so daß die saugaktive Oberfläche der Binde möglichst groß bleibt. Es kann also ein Sprühkleber oder in dünnen Fäden schmelzgeblasener Kleber verwendet werden. Auch ein Aufschweißen oder -siegeln durch Laser, Ultraschall oder Wärme ist denkbar.

In den Fig. 19 und 20 schließlich sind Ausführungsformen des erfindungsgemäß absorbierenden Artikels dargestellt, bei welchen der Saugkörper 205 eine plissierte Form aufweist. Die in den genannten Figuren gezeigten Artikel enthalten darüber hinaus auch eine plissierte Abdeckschicht 203, welche in Verbindungspunkten 202 mit einer äußeren Abdecklage 201 verbunden sind (Fig. 19). Die in Fig. 19 gezeigte Ausführungsform zeigt darüber hinaus eine flüssigkeitsundurchlässige Abdeckschicht 204, welche im Fachjargon auch als "Polybaffle" bezeichnet wird.

Der Saugkörper, welcher mindestens eine gewellte Bahn enthält, kann neben den vorstehend erwähnten Materialien Kardenvlies und Spinnvlies, beispielsweise auch eine luftgelegte Faserbahn, Laminate oder das weiter unten näher spezifizierte Material Coform enthalten. Zellstoff-Kunstfaser-Mischungen können problemlos bis zu 25 g/g Flüssigkeit aufnehmen. Zusätzlich zu einer saugfähigen Bahn kann der Saugkörper auch noch zusätzlich einen Superabsorber enthalten. Diese Superabsorber haben eine Flüssigkeitsaufnahmefähigkeit von etwa 1 bis 500 g/g physiologischer Kochsalzlösung. Für körpereigene Flüssigkeit beträgt das Aufnahmevermögen etwa 1 g/g bis 80 g/g.

Als flüssigkeitsdurchlässige Abdeckmaterialien sind insbesondere mehrlagige Laminate aus gelochten Folien und Spinnvliesen bzw. mehrlagige Laminate aus gelochten Folien und Kardenvliesen mit Flächengewichten von etwa 20 bis 200 g/m², vorzugsweise 30 bis 100 g/m², insbesondere 42 g/m², und/oder Dicken von 0,15 bis 4,0 mm, vorzugsweise 0,3 bis 1,5 mm, insbesondere 0,50 mm, geeignet.

Als Bestandteil des Saugkörpers eignen sich Materialien aus Kardenvlies, Spinnvlies, Bahnmaterial aus Zellstoff/Kunstfasermischung (Coform), eine verfestigte luftgelegte Zellstoffbahn (airlaid material), oder eine luftgelegte Zellstoff/Kunstfasermischung-Bahn (thermo fixiertes airlaid material). Weiterhin geeignet sind Prism- und Coform-Materialien, saugfähige Bahnen aus luftgelegtem Zellstoff, Zellstoff/Kunstfasermischungen oder Kunstfaservliese mit einem Flächengewicht von etwa 12 bis etwa 400 g/m², vorzugsweise 50 bis 150 g/m², insbesondere 100 g/m² und/oder Dicken von 0,1 bis 3 mm, vorzugsweise 0,2 bis 2 mm, insbesondere 1 mm.

Die körperseitige Abdecklage sollte vorzugsweise hydrophile Eigenschaften aufweisen. Mögliche weitere Transportschichten und/oder Materialien zwischen körperseitiger Abdecklage und Saugkörper können sowohl hydrophil als auch hydrophob sein.

Es versteht sich für den Fachmann von selbst, daß die plissierte Abdecklage 203 auch durch eine nichtplissierte Schicht ersetzt werden kann. Auch wenn nur zumindest ein Teil des Saugkörpers plissiert ist, werden bei einem erfindungsgemäßen Hygieneartikel die seitliche Auslaufsicherheit erhöht und eine bessere Längsverteilung der aufgenommenen Flüssigkeit erreicht. Des weiteren ist selbstverständlich, daß sowohl die flüssigkeitsdurchlässige Abdecklage als auch die flüssigkeitsundurchlässige Abdecklage bei einem absorbierenden Artikel mit einem zumindest teilweise gewellten Saugkörper aus den vorstehend näher beschriebenen Materialien aufgebaut sein können.

In Fig. 21 ist eine Ausführungsform des erfindungsgemäß absorbierenden Artikels dargestellt, bei welchem der Saugkörper 205 eine plissierte Form mit unterschiedlicher Höhe der Wellen aufweist. Die Wellenhöhe H₁ im Zentrum des absorbierenden Artikels beträgt dabei vorzugsweise maximal den zehnfachen Wert der Wellenhöhe H₂ in Randbereichen des Artikels.

Die erfindungsgemäß hergestellten längsgewellten Bahnen (plissierten Bahnen) wurden auf ultradünne Saugkörper aufgebracht. In Tabelle 2 sind die Leistungsdaten entsprechender absorbierender Artikel zusammengefaßt.

Die in Tabelle 2 spezifizierten Materialien Prism, Ekotec und Coform weisen im einzelnen die folgenden Eigenschaften auf:
- Prism:: Spezial-Spinnvlies mit hohem Volumen, einseitig oder zweiseitig behandelt; Flächengewichte von etwa 12 bis etwa 80 g/m². Prism 0,5 entspricht einem Flächengewicht von etwa 17 g/m². Prism 1,0 entspricht einem Flächengewicht von etwa 35 g/m².
- Ekotec:: high loft Kardenvlies, welches mittels heißer Luft getrocknet ist (hot through air dried), hohes Volumen; Flächengewichte von etwa 12 bis etwa 70 g/m².
- Coform:: Zellstoff/Polypropylenmischung, Zellstoff zerfasert mit gesponnenen PP-Fasern vergefestigt; Flächengewichte etwa 40 bis etwa 400 g/m².

Die einseitige oder zweiseitige Behandlung des Prism-Materials mittels eines Arivage-Verfahrens bewirkt eine bessere Flüssigkeitsaufnahme und ein schnelleres Eindringen der Flüssigkeit, speziell wenn das Material hydrophiliert wird.

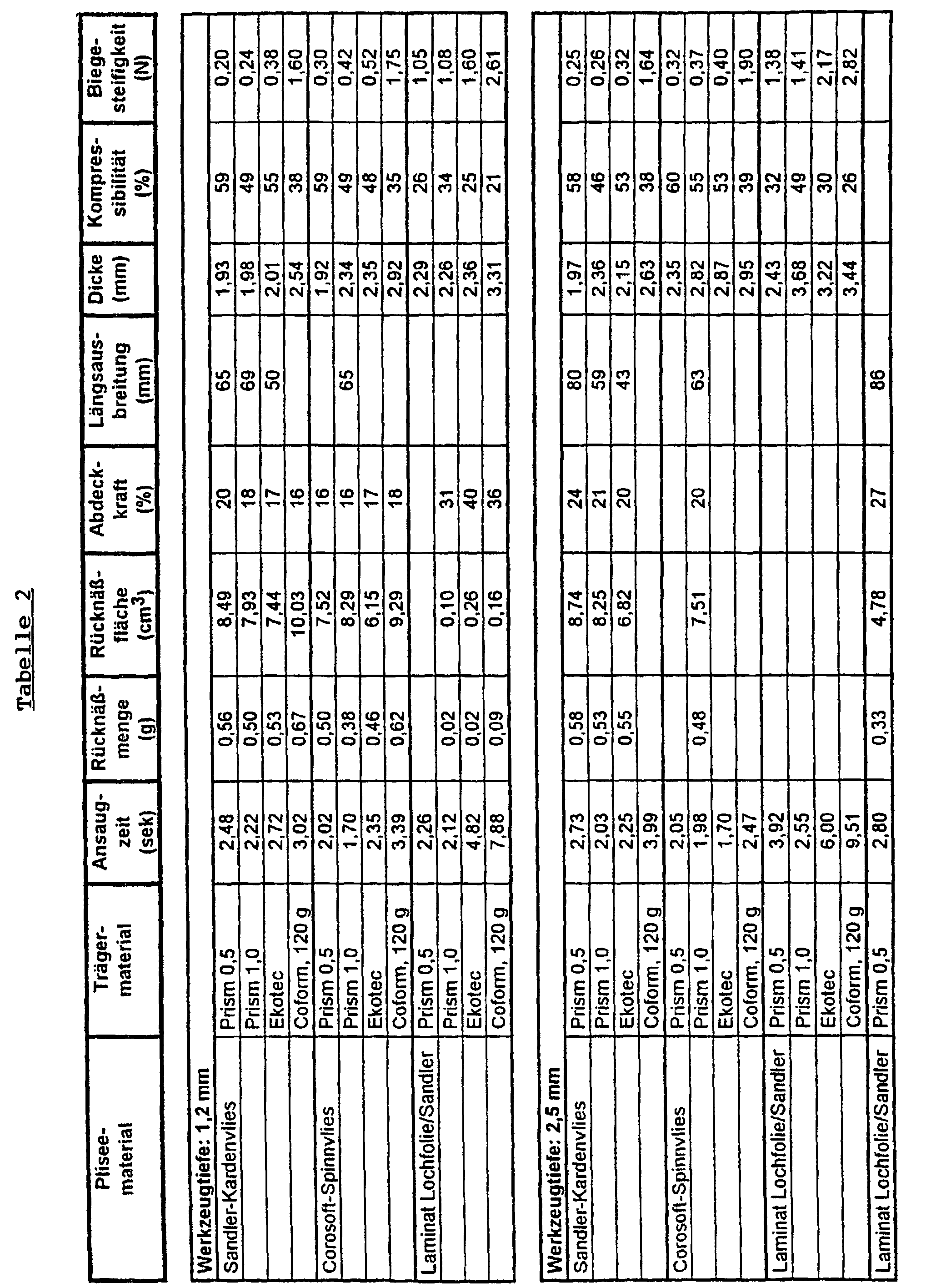

## Patentansprüche

1. Vorrichtung zur kontinuierlichen Erzeugung einer zumindest in Teilbereichen gewellten Bahn (1) aus dünnem, blattförmigem, und zumindest in Querrichtung zur Bahn dehnbarem Material, enthaltend
- ein Führungsbett (2), welches einen glatten Oberflächenbereich aufweist, an dem das Material anliegend vorbeiführbar ist, und welches in Bewegungsrichtung des Materials parallel oder schräg zueinander verlaufende Rillen aufweist, wobei jede Rille ausgehend von einer bezüglich der Bewegungsrichtung der Materialbahn stromaufwärt gelegenen Stelle in Richtung auf ein Austrittsende (4) des Führungsbetts (2) zunehmend tiefer in die Oberfläche des Führungsbetts (2) eingeschnitten ist und wobei die Oberseite des Führungsbetts (2) zumindest in dem Bereich, in dem die Rillen verlaufen, im wesentlichen eben ausgebildet ist,
- Niederhalter (5), welche den Rillen des Führungsbetts (2) gegenüberliegend derart angeordnet sind, daß sie die zwischen der Oberfläche des Führungsbetts (2) und den Niederhaltern (5) geführte Bahn zur Erzielung von Wellen in der Bahn in die Rillen des Führungsbetts (2) drängen,
sowie
- ein am Austrittsende (4) des Führungsbetts (2) angeordnetes Mittel, welches eine Rückbildung der ausgebildeten Wellen im wesentlichen verhindert.

2. Vorrichtung nach Anspruch 1, wobei das am Austrittsende (4) des Führungsbetts (2) angeordnete Mittel, welches die Rückbildung der ausgebildeten Wellen verhindert, unmittelbar an das Austrittsende des Führungsbetts anschließt.

3. Vorrichtung nach Anspruch 1 oder Anspruch 2, wobei die Niederhalter (5) eine Vorspannung aufweisen.

4. Vorrichtung nach einem der vorhergehenden Ansprüche, wobei die Niedehalter (5) ein freies, nicht geführtes oder gelagertes Ende aufweisen, welches in die Rillen eintauchbar ist.

5. Vorrichtung nach einem der vorhergehenden Ansprüche, wobei die Niederhalter (5) an dem Eintrittsende (3) des Führungsbetts (2) und/oder dem Austrittsende (4) des Führungsbetts (2) zugewandten Ende in senkrecht zum Führungsbett verlaufender Richtung verschiebbar gelagert sind.

6. Vorrichtung nach einem der vorhergehenden Ansprüche, wobei die Niederhalter (5) in zumindest einer senkrecht zum Führungsbett (2) verlaufenden Führungsfläche geführt sind.

7. Vorrichtung nach einem der vorstehenden Ansprüche, wobei die freien Enden der Niedehalter (5) im wesentlichen parallel zueinander abgebogen oder mit entsprechenden Ansätzen versehen sind, die in Bohrungen von zumindest einem endseitig gelegenen Führungselement aufnehmbar sind.

8. Vorrichtung nach einem der vorhergehenden Ansprüche, wobei alle Niederhalter bezüglich der Laufrichtung der Bahn vor bzw. nach dem Oberflächenbereich aufnehmbar sind.

9. Vorrichtung nach einem der vorhergehenden Ansprüche, **dadurch gekennzeichnet, daß** die Niederhalter (5) einen ferromagnetischen Werkstoff, vorzugsweise Stahl, enthalten.

10. Vorrichtung nach einem der vorhergehenden Ansprüche, wobei die Niederhalter (5) einen runden Querschnitt aufweisen.

11. Vorrichtung nach Anspruch 10, wobei die Niederhalter (5) einen Durchmesser von 0,1 bis 5 mm, vorzugsweise 1 bis 3 mm, insbesondere 2 mm, aufweisen.

12. Vorrichtung nach einem der vorhergehenden Ansprüche, wobei die Rillen einen Abstand von 1 bis 10 mm, vorzugsweise 2 bis 4 mm, aufweisen.

13. Vorrichtung nach einem der vorhergehenden Ansprüche, wobei diese Mittel aufweist, welche auf die Niederhalter einwirken, so daß diese mit einer vorgegebenen Kraft die Bahn (1) gegen das Führungsbett (2) drücken.

14. Vorrichtung nach Anspruch 13, wobei das Mittel so angeordnet ist, daß eine Krafteinwirkung auf die Niederhalter in einem Bereich erfolgt, welcher zwischen den Enden der Niederhalter (5) liegt.

15. Vorrichtung nach einem der Ansprüche 13 oder 14, wobei die Mittel zumindest ein Federelement (11), zumindest ein Gewicht, Druckluft, Vakuum und/oder zumindest einen Magneten enthalten.

16. Vorrichtung nach Anspruch 15, wobei der zumindest eine Magnet ein Permanentmagnet ist.

17. Vorrichtung nach Anspruch 15 oder 16, wobei der zumindest eine Magnet ein Elektromagnet (22) ist.

18. Vorrichtung nach einem der Ansprüche 15 bis 17, wobei der Magnet auf der den Niederhaltern gegenüberliegenden Seite des Führungsbetts (2) angeordnet ist.

19. Vorrichtung nach einem der Ansprüche 13 bis 18, wobei das Mittel eine Druckplatte (10) ist.

20. Vorrichtung nach Anspruch 19, wobei die Druckplatte aus Kunststoff ist.

21. Vorrichtung nach einem der vorhergehenden Ansprüche, wobei die Niederhalter (5) mit einer Kraft von 0,01 bis 0,1 Ncm⁻¹, vorzugsweise 0,02 bis 0,08 Ncm⁻¹, insbesondere 0,04 bis 0,06 Ncm⁻¹, belastet werden.

22. Vorrichtung nach einem der vorhergehenden Ansprüche, wobei das am Austrittsende (4) des Führungsbetts (2) angeordnete Mittel, welches eine Rückbildung der ausgebildeten Wellen verhindert, eine Einrichtung ist, um die gewellte Bahn mit zumindest einem bahnförmigsn Material (6) zu verbinden.

23. Vorrichtung nach Anspruch 22, wobei die Wellen der gebildeten gewellten Bahn im Bereich der Maxima und/oder Minima der Wellen mit dem bahnförmigen Material fixiert werden.

24. Vorrichtung nach Anspruch 19, **dadurch gekennzeichnet, daß** die Einrichtung zur Verbindung der gewellten Bahn mit einer Trägerbahn Walzen (12,13) aufweist, und daß die Niederhalter (5) in die Ausnehmungen einer profillierten Walze reichen.

25. Vorrichtung nach einem der Ansprüche 22 bis 24, wobei die Einrichtung zur Verbindung der gewellten Bahn (1) mit einem bahnförmigen Material (6) Mittel (15) aufweist, um auf das bahnförmige Material (6) und/oder die gewellte Bahn (1) ein Haftmittel aufzubringen.

26. Vorrichtung nach einem der vorhergehenden Ansprüche, wobei das Mittel, welches eine Rückbildung der ausgebildeten Wellen verhindert, eine Einrichtung ist, welche die gebildete gewellte Bahn durch thermische und/oder chemische und/oder physikalische Einwirkung in ihrer Form fixiert.

27. Vorrichtung nach einem der vorhergehenden Ansprüche, wobei das Führungsbett (2) so ausgebildet ist, daß die parallel zueinander verlaufenden Rillen im Mittelteil (17) des Führungsbetts (2) und weiter an den Randbereichen liegende Rillen stromabwärts zur Laufrichtung der Bahn jeweils trichterförmig beginnen.

28. Vorrichtung nach einem der vorhergehenden Ansprüche, wobei das Führungsbett (2) so ausgebildet ist, daß nicht die gesamte Breite mit Rillen versehen ist, sondern im Mittelteil und/oder in Randbereichen (19) des Führungsbetts (2) keine Rillen vorgesehen sind.

29. Vorrichtung nach einem der vorhergehenden Ansprüche, wobei die Einrichtung weiterhin eine Vorrichtung mit kämmenden Walzen (23,24) zur Prägung der erzeugten gewellten Bahn (1) aufweist, wobei die Einrichtung mit kämmenden Walzen dem Führungsbett (2) nachgeschaltet ist.

30. Verfahren zur kontinuierlichen Herstellung einer zumindest in Teilbereichen gewellten Bahn aus dünnem, blattförmigem und zumindest in Querrichtung zur Bahn dehnbarem Material, insbesondere mittels einer Vorrichtung gemäß einem der vorstehenden Ansprüche, **gekennzeichnet durch** folgende Schritte:
- Hindurchführen des Materials **durch** zumindest eine Vorrichtung, welche gegenseitig in Wirkverbindung stehende formgebende Elemente enthalten, und
- anschließendes Fixieren zumindest eines Bereichs der gewellten Bahn in einer Weise, daß zumindest der Abstand zweier benachbarter Wellen konstant gehalten wird.

31. Verfahren nach Anspruch 30, wobei das Material zwischen einem Führungsbett, das eine glatte Oberfläche und in Bewegungsrichtung des Materials parallel oder schräg zueinander verlaufende Rillen aufweist, wobei jede Rille ausgehend von einer bezüglich der Bewegungsrichtung des Materials stromaufwärts gelegenen Stelle in Richtung auf ein Austrittsende des Führungsbetts zunehmend tiefer in die Oberfläche des Führungsbetts eingeschnitten ist, und die Oberseite des Führungsbetts in dem Bereich, in dem die Rillen verlaufen, im wesentlichen eben verläuft, und Niederhaltern, welche den Rillen der Führungsbahn (2) gegenüberliegend derart angeordnet sind, daß sie eine zwischen der Oberfläche des Führungshaltersbetts (2) und den Niederhaltern (5) geführte Bahn zur Erzielung von Wellen in der Bahn in die Rillen des Führungsbetts (2) drängen, geführt wird.

32. Verfahren nach einem der Ansprüche 30 oder 31, wobei die Niederhalter mit einer vorgegebenen Kraft gegen die Bahn und das Führungsbett gedrückt wird.

33. Verfahren nach Anspruch 32, wobei die vorgegebene Kraft 0,01 bis 0,1 Ncm⁻¹, vorzugsweise 0,02 bis 0,08 Ncm⁻¹, insbesondere 0,04 bis 0,06 Ncm⁻¹, beträgt.

34. Verfahren nach Anspruch 32 oder 33, wobei die vorgegebene Kraft längs und/oder quer zur Laufrichtung der Bahn veränderlich eingestellt wird.

35. Verfahren nach einem der Ansprüche 32 bis 34, wobei die vorgebene Kraft mittels zumindest eines Federelement, zumindest eines Gewichts, Druckluft, Vakuum und/oder zumindest eines Magnets erzeugt wird.

36. Verfahren nach Anspruch 35, **dadurch gekennzeichnet, daß** der Magnet ein Permanentmagnet ist.

37. Verfahren nach einem der Ansprüche 35, wobei der Magnet ein Elektromagnet ist.

38. Verfahren nach einem der Ansprüche 30 bis 37, wobei die Fixierung der gewellten Bahn mittels Verbinden zumindest mit einem bahnförmigen Material erfolgt.

39. Verfahren nach Anspruch 38, wobei die längsgewellte Bahn durch ein Haftmittel mit dem bahnförmigen Material verbunden wird, wobei das Haftmittel vorzugsweise auf Wellenkämme bzw. Maxima der Wellen der gewellten Bahn aufgebracht wird oder auf die zulaufende Bahn an der der Maxima der Wellen gegenüberliegenden Orten aufgebracht wird.

40. Verfahren nach Anspruch 38 oder 39, wobei die gewellte Bahn durch thermische Einwirkung mit dem bahnförmigen Material verbunden wird.

41. Verfahren nach einem der Ansprüche 38 bis 40, wobei die gewellte Bahn durch Ultraschallschweißung mit dem bahnförmigen Material verbunden wird.

42. Verfahren nach einem der Ansprüche 38 bis 41, wobei die Anpreßkraft für die Verbindung der gewellten Bahn mit dem bahnförmigen Material über eine Strecke derart aufgebracht wird, daß der auf jede Welle wirkende Druck geringer ist als ein Druck, welcher die Welle deformiert.

43. Verfahren nach einem der Ansprüche 30 bis 42, wobei die Fixierung der gewellten Bahn durch thermische und/oder phsikalisch-chemische Einwirkung erfolgt.

44. Verfahren nach einem der Ansprüche 30 bis 42, wobei die Wellenbildung in Laufrichtung der Bahn im Mittelteil der Bahn beginnt und weiter an den Seiten liegende Wellen nacheinander ausgeformt werden.

45. Verfahren nach einem der Ansprüche 30 bis 44, wobei nicht die gesamte Bahn in Wellen gelegt wird, sondern in der Mitte und/oder am Rand ein Teil der Bahn unverformt bleibt.

46. Verfahren nach einem der Ansprüche 30 bis 45, wobei die erzeugte gewellte Bahn mittels kämmender Walzen nachgeprägt wird.

47. Verfahren nach einem der Ansprüche 30 bis 46, wobei die Materialbahn in Querrichtung um mindestens 10 % verformbar ist.

48. Verfahren nach Anspruch 47, wobei die Materialbahn zumindest in Querrichtung elastisch ist.

49. Verfahren nach einem der Ansprüche 30 bis 47, wobei als Materialbahn ein Kardenvlies, ein Spinnvlies, mehrlagige Laminate aus gelochten Folien und Spinnvliesen oder mehrlagige Laminate aus gelochten Folien und Kardenvliesen eingesetzt wird.

50. Verfahren nach Anspruch 49, wobei das Spinnvlies ein Flächengewicht zwischen 6 und 80 g/m², vorzugsweise 10 bis 30 g/m², insbesondere 16 g/m², und/oder eine Dicke von 0,05 bis 1,5 mm, vorzugsweise 0,1 bis 0,5 mm, besonders bevorzugt 0,1 bis 0,2 mm, insbesondere 0,13 mm, aufweist und das Kardenvlies ein Flächengewicht zwischen 6 und 80 g/m², vorzugsweise 10 bis 30 g/m², insbesondere 18 g/m² und/oder eine Dicke von 0,05 bis 1,5 mm, vorzugsweise 0,1 bis 0,5 mm, besonders bevorzugt 0,1 bis 0,2 mm, insbesondere 0,18 mm, aufweist.

51. Verfahren nach Anspruch 49, wobei die mehrlagigen Laminate ein Flächengewicht von 20 bis 200 g/m², vorzugsweise 30 bis 100 g/m², insbesondere 42 g/m² und/oder eine Dicke von 0,15 bis 4,0 mm, vorzugsweise 0,3 bis 1,5 mm, insbesondere 0,5 mm aufweisen.

52. Verfahren nach einem der Ansprüche 38 bis 52, **dadurch gekennzeichnet, daß** als bahnförmiges Material ein Spinnvlies mit hohem Volumen und/oder ein Kardenvlies mit hohem Volumen eingesetzt wird.

53. Absorbierender Artikel hergestellt durch das Verfahren nach einem der Ansprüche 30 bis 52, mit
- einer flüssigkeitsundurchlässigen Abdecklage,
- einer flüssigkeitsdurchlässigen Abdecklage, und
- einem zwischen flüssigkeitsundurchlässiger Abdecklage und flüssigkeitsdurchlässiger Abdecklage angeordneten Saugkörper,
- wobei die flüssigkeitsdurchlässige Abdecklage und/oder der Saugkörper eine gewellte Bahn enthalten, welche hergestellt wurde.

## Revendications

1. Dispositif de production continue d'une bande (1) ondulée au moins dans des régions partielles, en un matériau mince en forme de feuille et au moins extensible en direction transversale à la bande, comportant
- un lit de guidage (2) qui présente une région de surface lisse devant lequel le matériau peut passer à plat et qui présente en direction de mouvement du matériau des rainures s'étendant parallèlement ou en oblique les unes aux autres, chaque rainure, à partir d'un emplacement situé en amont par rapport à la direction de mouvement de la bande de matériau, étant entaillée de plus en plus profondément dans la surface du lit de guidage (2) en direction d'une extrémité de sortie (4) du lit de guidage (2), et la face supérieure du lit de guidage (2) étant réalisée sensiblement plane au moins dans la région dans laquelle s'étendent les rainures,
- des abaisseurs (5) qui sont agencés à l'opposé des rainures du lit de guidage (2) de telle sorte qu'ils pressent dans les rainures du lit de guidage (2) la bande guidée entre la surface du lit de guidage (2) et les abaisseurs (5) pour obtenir des ondulations dans la bande.
- un moyen agencé à l'extrémité de sortie (4) du lit de guidage (2), qui empêche sensiblement une rétrogradation des ondulations formées.

2. Dispositif selon la revendication 1, dans lequel le moyen agencé à l'extrémité de sortie (4) du lit de guidage, qui empêche la rétrogradation des ondulations formées, se raccorde directement à l'extrémité de sortie du lit de guidage.

3. Dispositif selon la revendication 1 ou la revendication 2, dans lequel les abaisseurs (5) présentent une précontrainte.

4. Dispositif selon l'une des revendications précédentes, dans lequel les abaisseurs (5) présentent une extrémité libre non guidée ni logée qui peut être plongée dans les rainures.

5. Dispositif selon l'une des revendications précédentes, dans lequel les abaisseurs (5) sont logés à déplacement à l'extrémité d'entrée (3) du lit de guidage (2) et/ou à l'extrémité tournée vers l'extrémité de sortie (4) du lit de guidage (2), en direction s'étendant perpendiculairement au lit de guidage.

6. Dispositif selon l'une des revendications précédentes, dans lequel les abaisseurs (5) sont guidés dans au moins une surface de guidage s'étendant perpendiculairement au lit de guidage (2).

7. Dispositif selon l'une des revendications précédentes, dans lequel les extrémités libres des abaisseurs (5) sont recourbées de manière sensiblement parallèle les unes par rapport aux autres ou sont pourvues de saillies correspondantes qui peuvent être reçues dans des perçages d'au moins un élément de guidage situé côté extrémité.

8. Dispositif selon l'une des revendications précédentes, dans lequel tous les abaisseurs peuvent être reçus en amont ou en aval de la région de surface par rapport à la direction de déroulement de la bande.

9. Dispositif selon l'une des revendications précédentes, **caractérisé en ce que** les abaisseurs (5) contiennent une substance ferromagnétique, de préférence de l'acier.

10. Dispositif selon l'une des revendications précédentes, dans lequel les abaisseurs (5) présentent une section transversale ronde.

11. Dispositif selon la revendication 10, dans lequel les abaisseurs (5) présentent un diamètre de 0,1 à 5 mm, de préférence 1 à 3 mm, en particulier 2 mm.

12. Dispositif selon l'une des revendications précédentes, dans lequel les rainures présentent un écartement de 1 à 10 mm, de préférence 2 à 4 mm.

13. Dispositif selon l'une des revendications précédentes, celui présentant des moyens qui agissent sur les abaisseurs de telle sorte que ceux-ci pressent avec une force prédéterminée la bande (1) contre le lit de guidage (2).

14. Dispositif selon la revendication 13, dans lequel le moyen est agencé de telle sorte que l'action de la force sur les abaisseurs a lieu dans une région qui est située entre les extrémités des abaisseurs (5).

15. Dispositif selon l'une ou l'autre des revendications 13 et 14, dans lequel les moyens contiennent au moins un élément faisant ressort (11), au moins un poids, de l'air comprimé, du vide et/ou au moins un aimant.

16. Dispositif selon la revendication 15, dans lequel ledit au moins un aimant est un aimant permanent.

17. Dispositif selon la revendication 15 ou la revendication 16, dans lequel ledit au moins un aimant est un électroaimant (22).

18. Dispositif selon l'une des revendications 15 à 17, dans lequel l'aimant est agencé sur le côté du lit de guidage (2) qui est opposé aux abaisseurs.

19. Dispositif selon l'une des revendications 13 à 18, dans lequel le moyen est une plaque de serrage (10).

20. Dispositif selon la revendication 19, dans laquelle la plaque de serrage est en matière plastique.

21. Dispositif selon l'une des revendications précédentes, dans lequel les abaisseurs (5) sont chargés d'une force de 0,01 à 0,1 Ncm⁻¹, de préférence de 0,02 à 0,08 Ncm⁻¹, en particulier de 0,04 à 0,06 Ncm⁻¹.

22. Dispositif selon l'une des revendications précédentes, dans lequel le moyen agencé à l'extrémité de sortie (4) du lit de guidage (2), qui empêche une rétrogradation des ondulations formées, est un mécanisme pour relier la bande ondulée à au moins un matériau en forme de bande (6).

23. Dispositif selon la revendication 22, dans lequel les ondulations de la bande ondulée formée sont fixées avec le matériau en forme de bande dans la région du maximum et/ou du minimum des ondulations.

24. Dispositif selon la revendication 19, **caractérisé en ce que** le mécanisme pour relier la bande ondulée avec une bande support présente des rouleaux (12, 13), et **en ce que** les abaisseurs (5) vont jusque dans les évidements d'un rouleau profilé.

25. Dispositif selon l'une des revendications 22 à 24, dans lequel le mécanisme pour relier la bande (1) ondulée avec un matériau (6) en forme de bande présente des moyens (15) pour appliquer un produit adhésif sur le matériau (6) en forme de bande et/ou sur la bande ondulée (1).

26. Dispositif selon l'une des revendications précédentes, dans lequel le moyen qui empêche une rétrogradation des ondulations formées est un agencement qui fixe dans sa forme la bande ondulée formée par action thermique et/ou chimique et/ou physique.

27. Dispositif selon l'une des revendications précédentes, dans lequel le lit de guidage (2) est réalisé de telle sorte que les rainures s'étendant parallèlement les unes aux autres commencent dans la partie médiane (17) du lit de guidage (2) et les rainures situées plus loin sur les régions de bord commencent en aval par rapport à la direction de la bande, respectivement en forme d'entonnoir.

28. Dispositif selon l'une des revendications précédentes, dans lequel le lit de guidage (2) est réalisé de telle sorte que la largeur n'est pas totalement pourvue de rainures, mais qu'il n'est pas prévu de rainures dans la partie médiane et/ou dans les régions de bord (19) du lit de guidage (2).

29. Dispositif selon l'une des revendications précédentes, dans lequel le mécanisme présente encore un dispositif avec rouleaux à engrènement (23, 24) pour estamper la bande ondulée (1) produite, le mécanisme avec des rouleaux à engrènement étant monté en aval du lit de guidage (2).

30. Procédé de fabrication continue d'une bande ondulée au moins dans des régions partielles, en un matériau mince en forme de feuille et au moins extensible en direction transversale à la bande, en particulier au moyen d'un dispositif selon l'une des revendications précédentes, **caractérisé en ce que** par les étapes suivantes :
- Passage du matériau à travers au moins un dispositif qui contient des éléments de façonnage en liaison active réciproque, et
- Fixation suivante d'au moins une région de la bande ondulée de manière à ce qu'au moins l'espacement entre deux ondulations voisines soit maintenu constant.

31. Procédé selon la revendication 30, dans lequel le matériau est guidé entre un lit de guidage qui présente une surface lisse et qui présente en direction de mouvement du matériau des rainures s'étendant parallèlement ou en oblique les unes par rapport aux autres, chaque rainure, à partir d'un emplacement situé en amont par rapport à la direction de mouvement de la bande de matériau, étant entaillée de plus en plus profondément dans la surface du lit de guidage en direction d'une extrémité de sortie du lit de guidage, et la face supérieure du lit de guidage s'étendant sensiblement à plat dans la région dans laquelle s'étendent les rainures, et des abaisseurs qui sont agencés à l'opposé des rainures du lit de guidage (2) de telle sorte qu'ils pressent dans les rainures du lit de guidage (2) une bande guidée entre la surface du lit de guidage (2) et les abaisseurs (5) pour obtenir des ondulations dans la bande.

32. Procédé selon l'une des revendications 30 ou 31, dans lequel les abaisseurs sont pressés avec une force prédéterminée contre la bande et le lit de guidage.

33. Procédé selon la revendication 32, dans lequel la force prédéterminée est de 0,01 à 0,1 Ncm⁻¹, de préférence de 0,02 à 0,08 Ncm⁻¹, en particulier de 0,04 à 0,06 Ncm⁻¹.

34. Procédé selon la revendication 32 ou la revendication 33, dans laquelle la force prédéterminée est réglée de manière variable le long de et/ou transversalement à la direction de déroulement de la bande.

35. Procédé selon l'une des revendications 32 à 34, dans lequel la force prédéterminée est engendrée par au moins un élément faisant ressort, au moins un poids, de l'air comprimé, du vide et/ou au moins un aimant.

36. Procédé selon la revendication 35, dans lequel ledit au moins un aimant est un aimant permanent.

37. Dispositif selon la revendication 35, dans lequel ledit au moins un aimant est un électroaimant.

38. Procédé selon l'une des revendications 30 à 37, dans lequel la fixation de la bande ondulée a lieu par liaison au moins avec un matériau en forme de bande.

39. Procédé selon la revendication 38, dans lequel la bande ondulée en longueur est reliée par un produit adhésif au matériau en forme de bande, le produit adhésif étant appliqué de préférence sur des crêtes d'ondulation ou sur les maxima des ondulations de la bande ondulée ou sur la bande arrivant aux endroits opposés au maxima des ondulations.

40. Procédé selon la revendication 38 ou 39, dans lequel la bande ondulée est reliée au matériau en forme de bande par effet thermique.

41. Procédé selon l'une des revendications 38 à 40, dans lequel la bande ondulée est reliée au matériau en forme de bande par soudage par ultrasons.

42. Procédé selon l'une des revendications 38 à 41, dans lequel la force de pressage pour la liaison de la bande ondulée avec le matériau en forme de bande est appliquée sur un trajet de telle manière que la pression agissant sur chaque ondulation est inférieure à une pression qui déforme l'ondulation.

43. Procédé selon l'une des revendications 30 à 42, dans lequel la fixation de la bande ondulée a lieu par action thermique et/ou physique-chimique.

44. Procédé selon l'une des revendications 30 à 42, dans lequel la formation d'ondulations commence en direction de déroulement de la bande dans la partie médiane de la bande et en ce que les ondulations se trouvant plus sur les côtés sont formées les unes après les autres.

45. Procédé selon l'une des revendications 30 à 44, dans lequel ce n'est pas toute la bande qui est mise en ondulation, mais une partie de la bande reste non déformée au milieu et/ou sur le bord.

46. Procédé selon l'une des revendications 30 à 45, dans lequel la bande ondulée produite est estampée ultérieurement au moyen de rouleaux à engrènement.

47. Procédé selon l'une des revendications 30 à 46, dans lequel la bande de matériau est déformable d'au moins 10 % en direction transversale.

48. Procédé selon la revendication 47, dans lequel la bande de matériau est élastique au moins en direction transversale.

49. Procédé selon l'une des revendications 30 à 47, dans lequel on utilise comme bande de matériau un voile de carde, une nappe non tissée, des stratifiés multicouches en feuilles perforées et des nappes non tissées ou des stratifiés multicouches en feuilles perforées et en voiles de carde.

50. Procédé selon la revendication 49, dans lequel la nappe non tissée présente un poids surfacique entre 6 et 80 g/m², de préférence 10 à 30 g/m², en particulier 16 g/m², et/ou une épaisseur de 0,05 à 1,5 mm, de préférence 0,1 à 0,5 mm, de préférence particulière de 0,1 à 0,2 mm, en particulier de 0,13 mm, et en ce que le voile de carde présente un poids surfacique entre 6 et 80 g/m², de préférence 10 à 30 g/m², en particulier 18 g/m², et/ou une épaisseur de 0,05 à 1,5 mm, de préférence 0,1 à 0,5 mm, de préférence particulière de 0,1 à 0,2 mm, en particulier de 0,18 mm.

51. Procédé selon la revendication 49, dans lequel les stratifiés multicouches présentent un poids surfacique 20 à 200 g/m², de préférence 30 à 100 g/m², en particulier 42 g/m², et/ou une épaisseur de 0,15 à 4,0 mm, de préférence 0,3 à 1,5 mm, en particulier de 0,5 mm.

52. Procédé selon l'une des revendications 38 à 52, **caractérisé en ce qu'**on utilise comme matériau en forme de bande une nappe non tissée de volume élevé et/ou un voile de carde de volume élevé.

53. Article absorbant comportant
- une couche de recouvrement imperméable aux liquides,
- une couche de recouvrement perméable aux liquides, et
- un corps d'aspiration agencé entre la couche de recouvrement imperméable aux liquides et la couche de recouvrement perméable aux liquides,
- la couche de recouvrement perméable aux liquides et/ou le corps d'aspiration contenant une bande ondulée qui a été réalisée par le procédé selon l'une des revendications 30 à 52.

## Claims

1. An apparatus for continuously producing a web (1) which is corrugated at least in partial sections thereof from a thin sheet material which is elastic at least in transverse direction of the web, comprising
- a guide bed (2) which comprises a smooth surface portion for the material to pass over in contacting manner and which comprises grooves extending parallel or inclined to each other in the direction of movement of the material, wherein each groove, starting from a point positioned upstream with respect to the direction of movement of the material web towards an outlet end (4) of the guide bed (2), is cut increasingly deeper into the surface of the guide bed (2), and wherein the upper side of the guide bed (2) is formed substantially planar at least in the portion in which said grooves extend,
- holding-down devices (5) arranged opposite to said grooves of the guide bed (2) so as to urge said web passing between the surface of said guide bed (2) and said holding-down devices (5) into the grooves of the guide bed (2) to obtain corrugations in the web,
and
- means arranged at the outlet end (4) of said guide bed (2) which substantially prevents the formed corrugations from springing back to the original condition.

2. The apparatus according to claim 1, wherein the means arranged at the outlet end (4) of the guide bed (2) preventing the formed corrugations from springing back is positioned directly after the outlet end of the guide bed.

3. The apparatus according to claim 1 or claim 2, wherein said holding-down devices (5) are biased.

4. The apparatus according to one of the preceding claims, wherein said holding-down devices (5) have a free end which is not guided or supported and which can be dipped into said grooves.

5. The apparatus according to one of the preceding claims, wherein the holding-down devices (5) are mounted at the end facing the inlet end (3) of the guide bed (2) and/or at the end facing the outlet end (4) of the guide bed (2) to be shiftable in a direction perpendicular to said guide bed.

6. The apparatus according to one of the preceding claims, wherein said holding-down devices (5) are guided in at least one guide face extending perpendicular to said guide bed (2).

7. The apparatus according to one of the preceding claims, wherein the free ends of said holding-down devices (5) are bent substantially parallel to each other or are provided with corresponding shoulders which can be received in bores of at least one guide element positioned at the end side.

8. The apparatus according to one of the preceding claims, wherein all holding-down devices, with respect to the direction of movement of the web, can be received forward and rearward, respectively, of the surface portion.

9. The apparatus according to one of the preceding claims, **characterized in that** said holding-down devices (5) contain a ferromagnetic material, preferably steel.

10. The apparatus according to one of the preceding claims, wherein said holding-down devices (5) have a round cross-section.

11. The apparatus according to claim 10, wherein said holding-down devices (5) have a diameter of 0.1 to 5 mm, preferably 1 to 3 mm, in particular 2 mm.

12. The apparatus according to one of the preceding claims, wherein said grooves have a distance of 1 to 10 mm, preferably 2 to 4 mm.

13. The apparatus according to one of the preceding claims, wherein said apparatus comprises means which acts on said holding-down devices such that said holding-down devices press said web (1) against said guide bed (2) with a predetermined force.

14. The apparatus according to claim 13, wherein said means are arranged in such a way that a force acts on said holding-down devices in a portion positioned between the ends of said holding-down devices (5).

15. The apparatus according to one of claims 13 or 14, wherein said means comprises at least one spring element (11), at least one weight, compressed air, vacuum and/or at least one magnet.

16. The apparatus according to claim 15, wherein the at least one magnet is a permanent magnet.

17. The apparatus according to claim 15 or 16, wherein the at least one magnet is an electromagnet (22).

18. The apparatus according to one of claims 15 to 17, wherein the magnet is arranged on the side of the guide bed (2) opposite to the holding-down devices.

19. The apparatus according to one of claims 13 to 18, wherein said means is a pressure plate (10).

20. The apparatus according to claim 19, wherein the pressure plate is made of plastic.

21. The apparatus according to one of the preceding claims, wherein a force of 0.01 to 0.1 Ncm⁻¹, preferably 0.02 to 0.08 Ncm^{-1,} in particular 0.04 to 0.06 Ncm⁻¹ is applied to said holding-down devices (5).

22. The apparatus according to one of the preceding claims, wherein the means arranged at the outlet end (4) of said guide bed (2) preventing the obtained corrugations from springing back to the original condition is a device for joining the corrugated web to at least one web-shaped material (6).

23. The apparatus according to claim 22, wherein the corrugations of the obtained corrugated web are fixed to the web-shaped material in the area of the maxima and/or minima of the corrugations.

24. The apparatus according to claim 19, wherein the device for joining the corrugated web to a backing web comprises rollers (12, 13) and said holding-down devices (5) extend into the recesses of a profiled roller.

25. The apparatus according to one of claims 22 to 24, wherein the means for joining the corrugated web (1) to a web-shaped material (6) comprises means (15) for applying an adhesive to the web-shaped material (6) and/or the corrugated web (1).

26. The apparatus according to one of the preceding claims, wherein said means preventing the obtained corrugations from springing back is a device for fixing the corrugated web formed in its shape by means of thermal and/or chemical and/or physical treatment.

27. The apparatus according to one of the preceding claims, wherein said guide bed (2) is configured in such a way that the grooves extending parallel to each other start in the central portion (17) of said guide bed (2), and grooves positioned closer to the edge portions start each in a funnel-shaped way downstream with respect to the direction of movement of the web.

28. The apparatus according to one of the preceding claims, wherein said guide bed (2) is configured in such a way that not the entire width is provided with grooves, but the central portion and/or edge portions (19) of the guide bed (2) are not provided with grooves.

29. The apparatus according to one of the preceding claims, wherein said device further comprises a means having meshing rollers (23, 24) for stamping the corrugated web (1) formed, said means which comprises meshing rollers being arranged downstream of said guide bed (2).

30. A process for continuously producing a web which is corrugated at least in partial sections thereof from a thin sheet material which is elastic at least in transverse direction of the web, in particular by means of an apparatus according to one of the preceding claims, **characterized by** the following steps:
- passing the material through at least one apparatus comprising forming elements operatively associated with each other, and
- subsequent fixing of at least one portion of the corrugated web in such a way that at least the distance between two adjacent corrugations is kept constant.

31. The process according to claim 30, wherein the material is passed between a guide bed having a smooth surface and comprising grooves extending parallel or inclined to each other in the direction of movement of the material, wherein each groove, starting from a point positioned upstream with respect to the direction of movement of the material towards an outlet end of the guide bed, is cut increasigly deeper into the surface of the guide bed, and the upper side of the guide bed extends substantially planar in the portion in which said grooves extend, with holding-down devices being arranged opposite to the grooves of said guide path (2) in such a way that they urge a web passed between the surface of the guide bed (2) and said holding-down devices (5) into the grooves of said guide bed (2) to obtain corrugations in the web.

32. The process according to one of claims 30 or 31, wherein said holding-down devices are pressed against said web and said guide bed with a predetermined force.

33. The process according to claim 32, wherein the predetermined force is 0.01 to 0.1 Ncm⁻¹, preferably 0.02 to 0.08 Ncm⁻¹, in particular 0.04 to 0.06 Ncm⁻¹.

34. The process according to claim 32 or 33, wherein said predetermined force is variably adjusted in a longitudinal and/or transverse direction of the direction of movement of the web.

35. The process according to one of claims 32 to 34, wherein said predetermined force is generated by means of at least one spring element, at least one weight, compressed air, vacuum and/or at least one magnet.

36. The process according to claim 35, **characterized in that** said magnet is a permanent magnet.

37. The process according to claim 35, wherein the magnet is an electromagnet.

38. The process according to one of claims 30 to 37, wherein the corrugated web is fixed by means of joining it to at least one web-shaped material.

39. The process according to claim 38, wherein the longitudinally corrugated web is joined to the backing web by means of an adhesive, which is applied to corrugation peaks or maxima of the corrugations of the corrugated web or to the supplied web at the points opposite to the maxima of the corrugations.

40. The process according to claim 38 or 39, wherein the corrugated web is joined to the web-shaped material by means of thermal treatment.

41. The process according to one of claims 38 to 40, wherein the corrugated web is joined to the web-shaped material by means of ultrasonic welding.

42. The process according to one of claims 38 to 41, wherein the pressing forc for joining the corrugated web to the web-shaped material is applied over a distance in a way that the pressure acting on each corrugation is less than a pressure which deforms the corrugation.

43. The process according to one of claims 30 to 42, wherein the corrugated web is fixed by means of thermal and/or physical-chemical treatment.

44. The process according to one of claims 30 to 42, wherein the corrugations start in the direction of movement of the web in the central portion of the web, and corrugations positioned closer to the sides are formed successively.

45. The process according to one of claims 30 to 44, wherein not the entire web is corrugated, but in the centre and/or at the edge a portion of the web remains without deformation.

46. The process according to one of claims 30 to 45, wherein the produced corrugated web is subsequently stamped by means of meshing rollers.

47. The process according to one of claims 30 to 46, wherein the material web is deformable in transverse direction by at least 10 %.

48. The process according to claim 47, wherein the material web is elastic at least in transverse direction.

49. The process according to one of claims 30 to 47, wherein a card nonwoven, a spunbonded nonwoven, multi-layer laminates of perforated foils and spunbonded nonwovens or multi-layer laminates of perforated foils and card nonwovens are used as the material web.

50. The process according to claim 49, wherein the spundbonded nonwoven has a basis weight between 6 and 80 g/m², preferably 10 to 30 g/m², in particular 16 g/m², and/or a thickness of 0.05 to 1.5 mm, preferably 0.1 to 0.5 mm, more preferred 0.1 to 0.2 mm, in particular 0.13 mm, and the card nonwoven has a basis weight between 6 and 80 g/m², preferably 10 to 30 g/m², in particular 18 g/m² and/or a thickness of 0.05 to 1.5 mm, preferably 0.1 to 0.5 mm, more preferred 0.1 to 0.2 mm, in particular 0.18 mm.

51. The process according to claim 49, wherein the multi-layer laminates have a basis weight of 20 to 200 g/m², preferably 30 to 100 g/m², in particular 42 g/m², and/or a thickness of from 0.15 to 4.0 mm, preferably 0.3 to 1.5 mm, in particular 0.5 mm.

52. The process according to one of claims 38 to 52, **characterized in that** a spunbonded nonwoven having a high volume and/or a card nonwoven having a high volume is used as the web-shaped material.

53. An absorbent article made by means of the process according to one of claims 30 to 52, comprising
- a liquid-impermeable cover sheet,
- a liquid-permeable cover sheet, and
- an absorbent body arranged between the liquid-impermeable cover sheet and the liquid-permeable cover sheet,
- wherein said liquid-permeable cover sheet and/or said absorbent body comprise a corrugated web.
